(19) Europäisches Patentamt
European Patent Office
Office européen des brevets

(11) **EP 4 209 502 A1**

(12) **EUROPEAN PATENT APPLICATION**

(43) Date of publication:
**12.07.2023 Bulletin 2023/28**

(51) International Patent Classification (IPC):
**C07K 14/415** *(2006.01)* **C12N 15/82** *(2006.01)*
**A01H 5/00** *(2018.01)*

(21) Application number: **22150603.3**

(22) Date of filing: **07.01.2022**

(52) Cooperative Patent Classification (CPC):
**C12N 15/8245; C07K 14/415; C12N 15/8273**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **KWS SAAT SE & Co. KGaA
37574 Einbeck (DE)**
• **Südzucker AG
68165 Mannheim (DE)**
• **Technische Universität Kaiserslautern
67663 Kaiserslautern (DE)**

(72) Inventors:
• **Khan, Azkia
Kaiserslautern (DE)**

• **Neuhaus, Ekkehard
Kaiserslautern (DE)**
• **Pommerrenig, Benjamin
Kulmbach (DE)**
• **Ludewig, Frank
Einbeck (DE)**

(74) Representative: **Grünecker Patent- und
Rechtsanwälte
PartG mbB
Leopoldstraße 4
80802 München (DE)**

Remarks:
The complete document including Reference
Table(s) and the Sequence Listing(s) can be
downloaded from the EPO website

(54) **FRUCTOSE SENSING AND SIGNALING IN PLANTS**

(57) The invention is directed to fructose sensing and signaling mechanisms in plants for improving plant properties. In particular, the invention concerns a vector or mobile genetic element, comprising a nucleic acid molecule, wherein the nucleic acid molecule encodes a tonoplast proton-fructose symporter. Further the invention concerns a host cell, a plant, a seed, a method of producing a plant, a method of increasing the yield of a plant storage compound, a method of increasing drought tolerance in plants, use of a tonoplast proton-fructose symporter in a plant to increase the concentration of a plant storage compound or a method of selecting a plant with increased fructose levels.

EP 4 209 502 A1

**Description**

**BACKGROUND OF THE INVENTION**

The present invention is concerned with providing and generating plants with beneficial characteristics. In particular, the invention is concerned with providing and generating crop plants that produce increased amounts of biomass and/or increased amounts of desired plant storage compound(s), e.g. sugars, such as sucrose, starch, lipids and/or proteins, and/or have an increased tolerance to adverse environmental conditions, such as drought.

The present inventors have surprisingly found that increasing the fructose concentration in the cytosol of a plant changes the sugar signaling in the plant, for instance in the leaf of the plant, which leads to a change in the long-distance transport of sugars, such as sucrose in the phloem of the plant. Particularly, the long-distance transport in the plant changes so that more sugar is transported from the plant sites of carbohydrate synthesis (source) to the plant sites of storage (sink) of the plant storage compounds. In plant organs that serve as the sink of plant storage compounds, such compounds may be converted into other plant storage compounds. For example, sugars may be converted into starch or cellulose in the plant organ. Plant storage compounds may include, without limitation, sugars such as sucrose, starch, proteins and/or lipids. In addition, the inventors have found that increasing the concentration of fructose in the cytosol of a plant, in particular by overexpressing a tonoplast proton-fructose symporter, e.g. EDRL4, increases the biomass of the plant.

The inventors' observations strongly suggest that an increase in cytosolic fructose levels can be sensed by the plant in different plant compartments, thereby leading to changes in long-distance transport of sugar, which has a positive effect on biomass production, drought resistance and in particular plant storage compound e.g. sucrose, accumulation in plant organs, such as a fruit, a seed, a root or a storage organ. Increasing the amount of sucrose in sucrose storage compartments of a plant is particularly desirable in the case of crop plants, such as sugar beets. Accordingly, as the skilled person will appreciate, any means of increasing the fructose concentration in the cytosol of a plant may be used to achieve the beneficial effects described herein.

**SUMMARY OF THE INVENTION**

In one aspect, the invention concerns a vector or mobile genetic element, comprising a nucleic acid molecule, wherein the nucleic acid molecule encodes a tonoplast proton-fructose symporter. In particular, the nucleic acid molecule is selected from the group consisting of: a) a nucleic acid molecule having at least 50% nucleic acid sequence identity with a nucleic acid sequence selected from the group consisting of any one of the sequences according to SEQ ID NO: 40, 39, 1, and 129-152, b) a nucleic acid molecule having a nucleic acid sequence that is complementary to or hybridizes under stringent conditions with the nucleic acid molecule according to a), and c) a nucleic acid molecule having a nucleic acid sequence that encodes a protein having at least 50% amino acid sequence identity with an amino acid sequence selected from the group consisting of any one of the sequences according to SEQ ID NO: 58, 57, 22, and 77-128.

In a further aspect the invention includes a host cell comprising the vector or mobile genetic element.

The invention also includes a plant or a part of a plant comprising at least one host cell as described herein. The plant of the inventions may be genetically modified. The plant may be a transgenic plant.

In one embodiment provided is a plant, wherein the plant has been genetically modified so that the plant comprises elevated levels of fructose in the cytosol relative to the wild type plant from which it is derived. The plant of the invention may overexpress a tonoplast proton-fructose symporter relative to the wild type plant from which it is derived.

In some embodiments, the plant of the invention is characterized as follows:

i) the plant comprises a modified promotor of an endogenous gene encoding the overexpressed tonoplast proton-fructose symporter so that the tonoplast proton-fructose symporter is homologously overexpressed,

ii) the plant comprises at least one additional copy of a nucleic acid encoding the overexpressed tonoplast proton-fructose symporter so that the tonoplast proton-fructose symporter is homologously overexpressed, or

iii) the plant comprises a heterologous nucleic acid encoding a heterologous tonoplast proton-fructose symporter so that the tonoplast proton-fructose symporter is heterologously overexpressed.

In some embodiments of the invention, the plant comprises the vector or mobile genetic element of the invention. The vector or the mobile genetic element may be introduced into the plant as described herein, in the cited references and by any other means known to the skilled person.

The invention also includes a seed of a plant according to the invention. The seed may be planted to provide a plant according to the invention. The plant may be grown to solve the technical problems described herein, e.g. to increase the yield of a plant storage compound, to increase the biomass, to increase the concentration of the plant storage compound in at least one plant organ or to increase the drought tolerance of a plant.

In a further aspect, the invention includes a method of producing a plant, comprising the following steps: a) genetically modifying at least one plant cell so that at least one plant cell is produced that comprises elevated levels of fructose in the cytosol relative to the wild type plant cell from which it is derived; and b) regenerating a plant from the genetically modified plant cell of step a), wherein the plant comprises elevated levels of fructose in the cytosol relative to the wild type plant from which is derived.

In some embodiments, genetically modifying the at least one plant cell, e.g. in step a), comprises:

i) modifying the promoter of an endogenous gene encoding a tonoplast proton-fructose symporter so that the tonoplast proton-fructose symporter is overexpressed,

ii) introducing into the plant cell at least one additional copy of a nucleic acid encoding a tonoplast proton-fructose symporter so that the tonoplast proton-fructose symporter is homologously overexpressed, or

iii) introducing into the plant cell a heterologous nucleic acid encoding a heterologous tonoplast proton-fructose symporter so that the tonoplast proton-fructose symporter is heterologously overexpressed.

In some embodiments, the vector or mobile genetic element of the invention is introduced into the at least one plant cell.

The invention also includes a method of increasing the yield of a plant storage compound, comprising increasing the concentration of fructose in the cytosol of the plant. In some embodiments, the plant that produces the plant storage compound is produced as described herein. In some embodiments, the plant that produces the plant storage compound is a plant as described herein. The concentration of fructose in the cytosol of a plant can be increased as described herein.

The plant storage compound may be selected from a sugar, preferably sucrose, starch, a protein or a lipid. In particular, the sugar may be a sugar as present in sugar beet, soybeans or tomato fruit, the lipid may be a lipid present in sunflower seed or oilseed rape, the protein may be a protein present in soybeans. Preferably, the plant storage compound is sucrose. The method of increasing the yield of a plant storage compound may comprise the following:

i) the biomass of at least one plant organ that comprises the plant storage compound is increased, preferably the plant organ is a fruit, a seed, a root or a storage organ; or

ii) the concentration of the plant storage compound in at least one plant organ is increased, preferably the plant organ is a fruit, a seed, a root or a storage organ.

In some embodiments, the yield of a plant storage compound is increased by increasing the biomass of at least one plant organ, such as a fruit, a seed, a root or a storage organ. In some embodiments, the yield of a plant storage compound is increased by increasing the concentration of the plant storage compound in at least one plant organ, such as a fruit, a seed, a root or a storage organ.

In a further aspect, the invention provides a method of increasing drought tolerance in plants, comprising increasing the concentration of fructose in the cytosol of the plant. The method may comprise:

i) the plant that has increased drought tolerance is produced according to the methods of the invention; and/or

ii) the plant that has increased drought tolerance is a plant according to the invention.

The plant may be a crop plant. In some embodiments, the plant is selected from the group consisting of sugar beet (Beta vulgaris), cabbage (Brassica rapa), oilseed rape (Brassica napus), sunflower (Helianthus annuus), potato (Solanum tuberosum), corn (Zea mays), rice (Oryza sp., e.g. Oryza sativa), barley (Hordeum vulgare), rye (Secale cereale) or sorghum (Sorghum sp., e.g. Sorghum bicolor), preferably the plant is sugar beet.

In a preferred embodiment, the plant is sugar beet, the plant organ is the sugar beet taproot and the plant storage compound is sucrose.

The inventions also provides a use of a tonoplast proton-fructose symporter in a plant to increase the concentration of a plant storage compound in a plant organ, optionally the plant storage compound is selected from a sugar, preferably sucrose, starch, a protein or a lipid, and the plant is selected from the group consisting of sugar beet (Beta vulgaris), cabbage (Brassica rapa), oilseed rape (Brassica napus), sunflower (Helianthus annuus), potato (Solanum tuberosum), corn (Zea mays), rice (Oryza sp., e.g. Oryza sativa), barley (Hordeum vulgare), rye (Secale cereale) or sorghum (Sorghum sp., e.g. Sorghum bicolor), preferably the plant is sugar beet and the plant organ is the sugar beet taproot.

The invention also provides a method of selecting a plant with increased fructose levels in the cytosol of the plant, comprising the steps:

i) growing a plant;

ii) measuring the fructose level in at least one plant cell of the plant;

iii) comparing the measured fructose level to at least one fructose reference level; and

iv) selecting the plant with increased fructose levels relative to the at least one reference level.

The reference level may be the fructose level in the cytosol of a reference plant. The reference plant may be a plant from which the plant is derived. The reference plant may be a plant of the same species. For instance, the reference plant may be a wild type plant from which the plant is derived if the plant has been genetically modified. In some embodiments, the plant is a plant of the invention, e.g. comprising a vector or genetic element as described herein. The plant may also overexpress a tonoplast proton-fructose symporter as described herein. In some embodiments, the reference fructose level is determined in the same plant organ and/or same plant cell type.

## BRIEF DESCRIPTION OF DRAWINGS

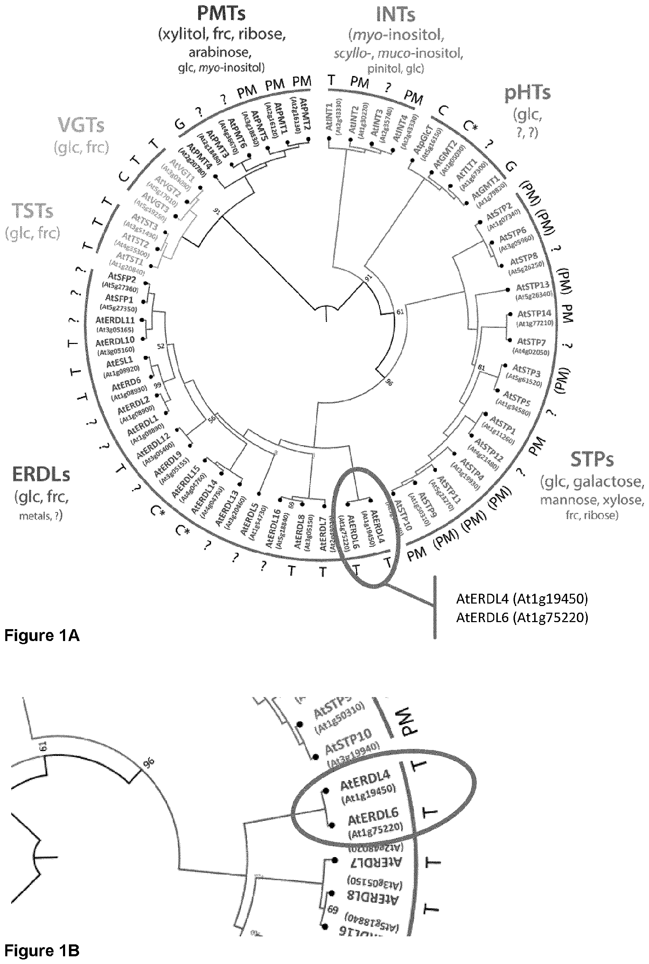

**Figure** 1. Phylogenetic tree of the MST transporter family (Figure 1A). The figure is taken from Pommerrenig et al. (2018). Phylogenetic tree of the 53 Arabidopsis MST family proteins based on their nucleotide sequences. Numbers at nodes show node support values. Only values <100 are shown. MST subfamilies are abbreviated as follows: PMTs, POLYOL MONOSACCHARIDE TRANSPORTERs; VGTs, VACUOLAR GLUCOSE TRANSPORTERs; TSTs, TONOPLAST SUGAR TRANSPORTERs; ERDLs, EARLY-RESPONSE TO DEHYDRATION SIX-LIKE proteins; STPs, SUGAR TRANSPORTERs; pHTs, PLASTIDIC HEXOSE TRANSPORTERs; INTs, INOSITOL TRANSPORTERs. Names of individual transport proteins are given together with their Arabidopsis identifier (in parentheses). Letters outside of the circle indicate the subcellular localization of the proteins: PM, plasma membrane; T, tonoplast; C, chloroplast; G, Golgi apparatus. Asterisks indicate predicted chloroplast localization due to identification of chloroplast transit peptide sequences (ChloroP 1.1; Emanuelsson et al. 1999). Letters in parentheses indicate presumptive localization, e.g. when localization has been shown in heterologous systems. Question marks indicate unknown subcellular localization. Tested and putative transport substrates are listed for each subfamily. A larger font size indicates higher affinity of subfamily members for substrate. The MST nucleotide sequences were obtained from the aramemnon database (Schwacke et al. 2003). Multiple sequence alignment for all MST sequences was built using Clustal Omega (Sievers et al. 2011). Bayesian phylogenetic analysis was performed with MrBayes version 3.2.6 (Ronquist and Huelsenbeck 2003) using the HKY + I + G model. MrBayes was run by conducting two parallel Metropolis-coupled Monte Carlo Markov chain analyses with four chains for 2 million generations. The SD of split frequencies was <0.01. The tree file was visualized using FigTree v. 1.4.3. Figure 1B shows a blow-up of the portion including the AtERDL4 gene.

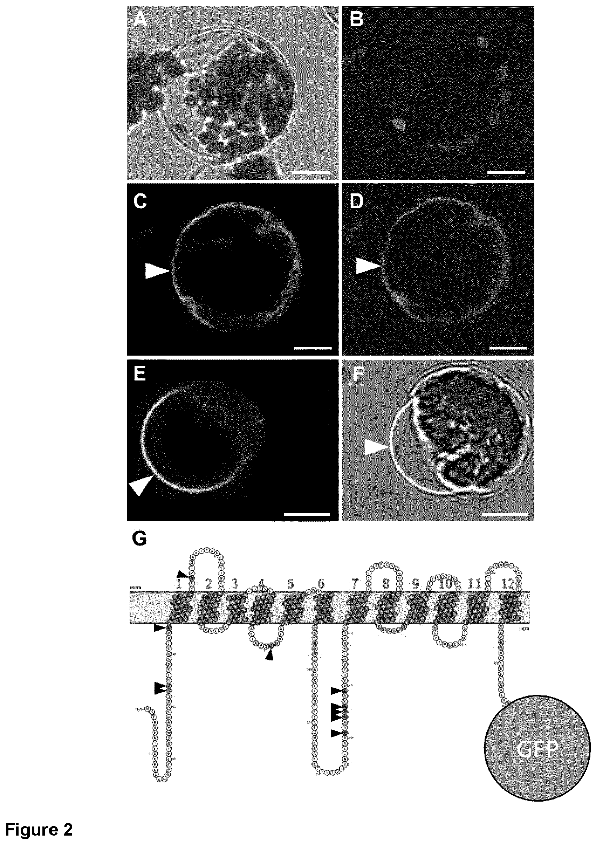

**Figure 2.** Tonoplast localization of an N-terminal fusion of ERDL4 with GFP. A-F) Confocal images from tobacco mesophyll protoplasts transformed with an PromoterUBQ10-ERDL4-GFP construct. Scale bars are 10 $\mu$m A-F) Images of a tobacco protoplast expressing the ERDL4-GFP fusion. Different confocal channels of the same protoplast are shown. A) Bright-field image B) Chlorophyll-derived auto-fluorescence indicating position of chloroplasts (spheres) C) GFP-derived fluorescence indicating position of the ERDL4-GFP fusion protein D) overlay of chlorophyll and GFP fluorescence. E and F) images of a lysed protoplast expressing the ERDL4-GFP fluorescence E) GFP-derived fluorescence at the vacuolar membrane (the tonoplast) F) overlay picture of GFP fluorescence and bright field image. White triangles in C-F point towards the tonoplast G) schematic depiction of 2D-structure of the ERDL4 protein fused to GFP at the C-terminus of the ERDL4 protein. Different numbers within the protein structure indicate different transmembrane helices in the ERDL4 protein. Black triangles point towards predicted phosphorylation sites, serine or threonine residues.

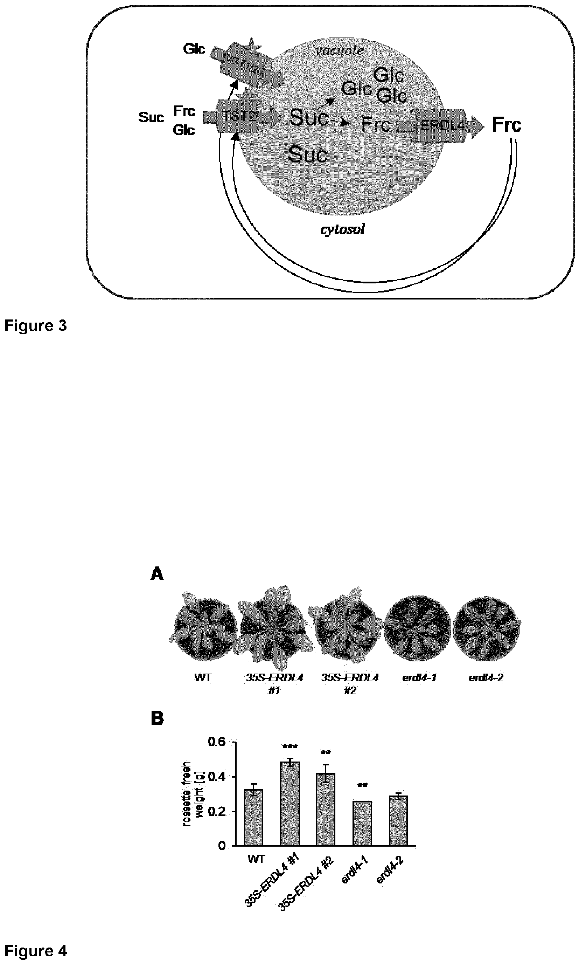

**Figure 3.** Schematic representation of ERDL4 localization and proposed function. TST2 and VGT1/2-dependent transport of sugars glucose (glc), fructose (frc) and sucrose (suc) is stimulated by cytosolic fructose. Vacuolar frc derived by TST import or vacuolar suc hydrolysis is transported via ERDL4 into the cytosol and activates TST2 and VGT1/2 activity (indicated by stars above schematic TST2 and VGT1/2 transporters). Because of high specificity of ERDL4 for frc, in sum high concentrations of suc and glc, but not frc accumulate in vacuoles.

**Figure 4.** Phenotypes and fresh weight of different erdl4 mutant and ERDL4-overexpressing plants. A) Phenotypes of four-week-old WT, 35S-ERDL4, and erdl4 knock-out plants grown in soil substrate under short day conditions. B) Quantification of shoot biomass on fresh weight basis of plants depicted in (A). Bars show means from n=12 independent plants $\pm$ SE. Asterisks indicate significant differences to WT according to Students t-test (* $p < 0.05$).

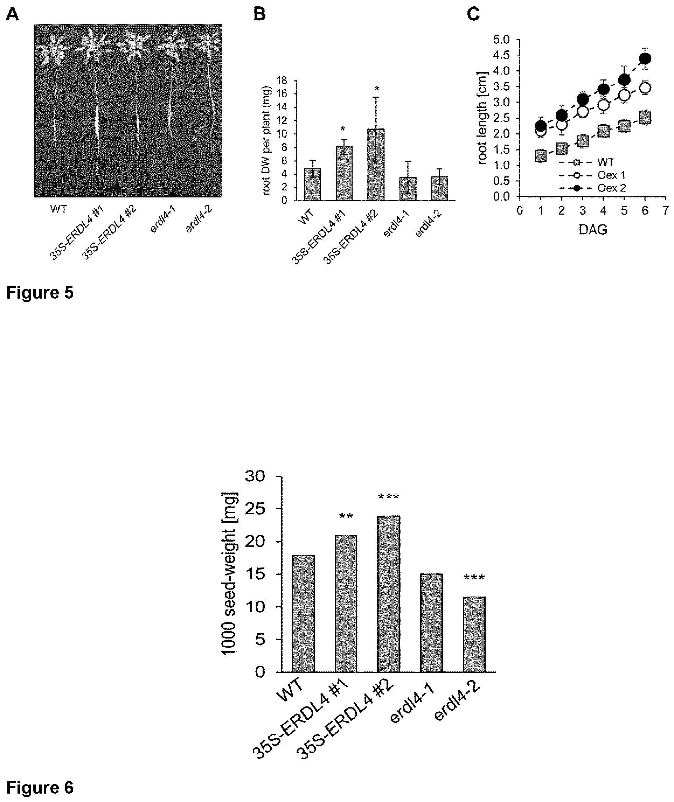

**Figure 5.** Characterization of root parameters of different erdl4 mutant and ERDL4-overexpressing plants. A) Exemplary pictures of rosettes and roots from five-week-old WT, 35S-ERDL4, and erdl4 knock-out plants grown in hydroponic culture. ERDL4 overexpressing plants have longer roots with higher biomass. B) Dry biomass of roots from plants depicted in (A). Bars show means from n=6 roots $\pm$ SE. Asterisk indicate significant differences to WT according to Students t-test. C) Length of primary roots from WT and 35S-ERDL4 plants measured over a time course of six days.

**Figure 6.** Thousand Seed weight is increased in ERDL4 over-expressors. 1000-Seed-weight of dry mature seeds from WT, erdl4-knock-out mutants and ERDL4-overexpessing plants. Bars represent means from 1000-Seed weights of n=6 plants per line. Asterisks indicate significant differences according to Students t-test (** = p<0.01; *** = p < 0.001).

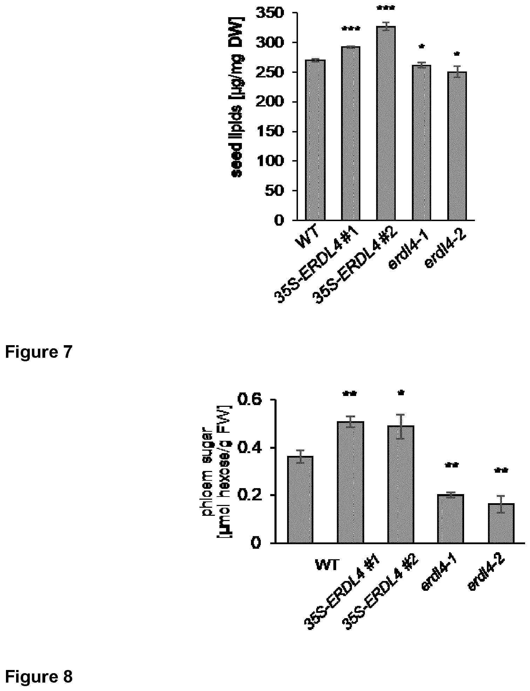

**Figure 7.** Seed lipid content of dry mature seeds from erdl4-knock-out mutants and ERDL4-overexpessing plants. Bars represent means from n=6 plants per line. Asterisks indicate significant differences according to Students t-test (* = p < 0.05; ** = p<0.01; *** = p < 0.001).

**Figure 8.** Increased phloem flux of sugar from detached leaves of ERDL4-overexpressors. Sugar measured as hexose equivalents in phloem exudates collected from detached leaves of five-week-old WT, 35S-ERDL4, and erdl4 knock-out plants. Bars are means from at least n=12 leaves $\pm$ SE. Asterisks indicate significant differences to WT according to Students t-test.

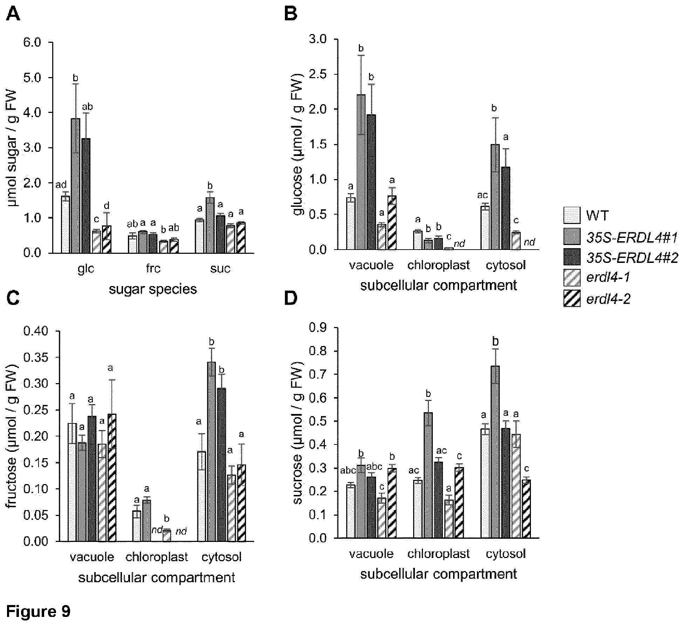

**Figure 9.** Total and subcellular sugar accumulation in WT, ERDL4-overexpressing and mutant plants. A) Contents of glucose (glc), fructose (frc), sucrose (suc) in shoots. B-D) subcellular contents of glucose (B), fructose (C), sucrose (D) in vacuoles, chloroplast, and cytosolic fractions of shoots from WT, ERDL4-overexpressing and mutant plants as obtained by non-aqueous fractionation. Bars represent means from n=5 plants $\pm$ SE. Different letters above bars denote significant differences according to One-Way ANOVA with post-hoc Tukey test (p < 0.05). nd = no quantifiable amount detected.

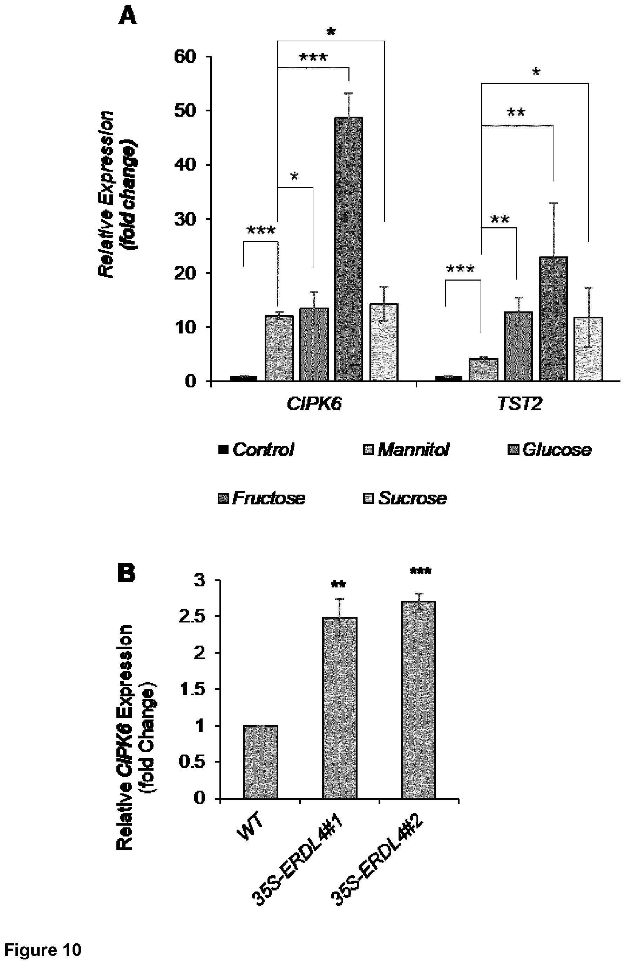

**Figure 10.** CIPK6 expression is increased by fructose as well as in ERDL4-overexpressing lines. A) Relative expression of CIPK6 (At4g30960) and TST2 (At4g35300) in response to different sugars or mannitol. The sugar treatment was performed by incubating leaf discs excised from Arabidopsis thaliana source leaves in half-strength MS medium supplemented without (control) or with 2% of the respective sugar or mannitol. Expression was quantified with RT-qPCR and normalized to expression of AtACT2. B) Relative expression of CIPK6 (At4g30960) in WT and two 35S-ERDL4 lines. A-B) Bars represent means of n=4 replicates $\pm$ SE. Asterisks indicate significant differences according to Students t-test (* = p < 0.05; ** = p < 0.005; *** = p < 0.001).

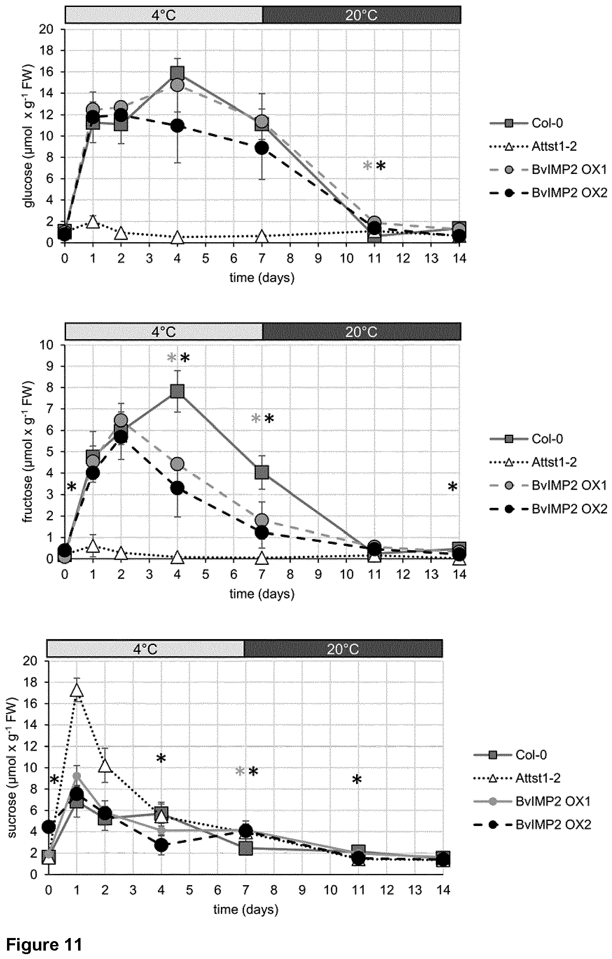

**Figure 11.** Cold acclimation and de-acclimation kinetics of sugar contents in leaves of Arabidopsis thaliana. The time course was recorded for Col-0, tst1-2 double knock-out, and two BvIMP2 (=BvERDL4;2) overexpressing lines under cold stress (4°C) and de-acclimation (20°C). A) Glucose content B) Fructose content C) Sucrose content. Each time point represents the mean value from at least three plants per line. Error bars indicate SEs. Asterisks denote significant changes of BvIMP2 overexpressing lines in comparison to the WT at a given time point. Different colors of asterisks indicate affiliation with the respective line (grey = BvIMP2 OX1, black = BvIMP2 OX2).

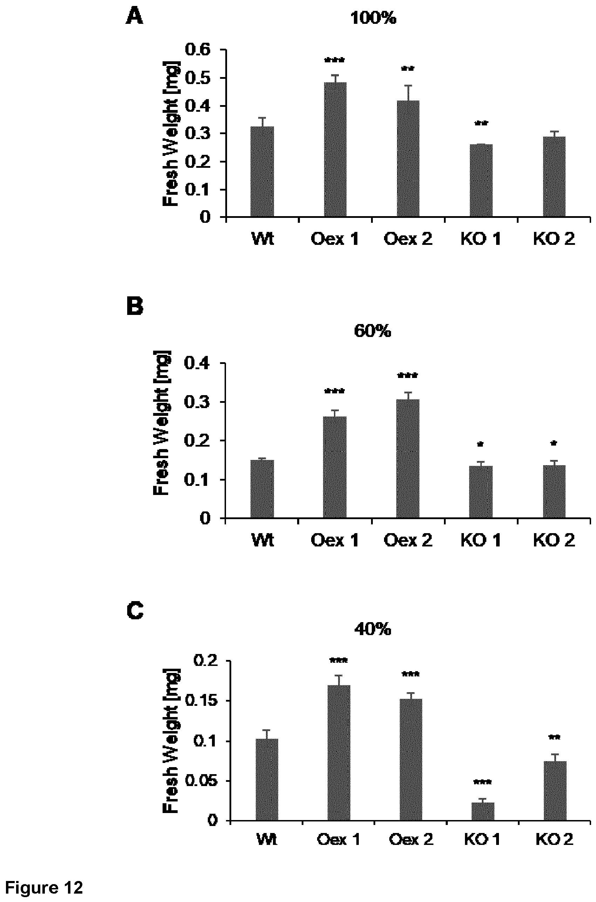

**Figure 12.** Fresh weight of plants grown under control or drought conditions. Drought stress was applied based on field capacity (FC) of the soil substrate (A) 100% FC = control, well-watered condition; (B) 60% FC = mild drought stress, (C) 40% FC = severe drought stress to Arabidopsis thaliana Col-0 (Wt, control), two ERDL4-overexpressing lines (Oex1, Oex2) and erdl4-knock out lines (KO 1, KO2). Plants were subjected to stress after seven days of growth under 100% FC and grown under the respective FC for 3.5 weeks. Bars represent means of at least n=6 plants $\pm$ SE. Asterisks denote significant differences in comparison to the WT according to Students t-test (* = p < 0.05; ** = p < 0.005; *** = p < 0.001).

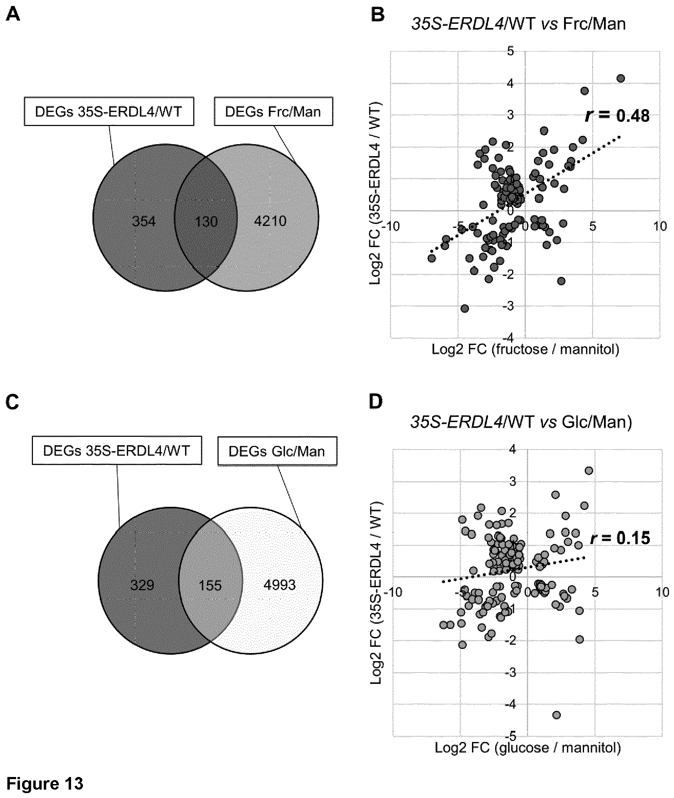

**Figure 13.** Venn-diagram and correlation analysis between differentially expressed genes (DEGs) from ERDL4-overexpressing and WT plants and from fructose (Frc) or glucose (Glc) and mannitol (Man = control) incubated leaf discs. A) and C) show Venn-diagrams of DEGs. Numbers within circles represent number of DEGs (significant at p < 0.001 according to double-sided t-test of three replicates). There were 484 35S-ERDL4-dependent DEGs, 4340 Frc-dependent DEGs, and 5148 Glc-dependent DEGs. A) Diagram showing overlap of DEGs from 35S-ERDL4 and Frc-treatment. There were 130 DEGs regulated by both ERDL4-overexpression and Frc. B) Correlation between relative expression (based on log2-fold-change) of 130 DEGs between 35S-ERDL4 (against WT) and Frc (against Man). r = Pearson coefficient calculated from ERDL4/WT and Frc/Man matrices. C) Diagram showing overlap of DEGs from 35S-ERDL4 and Glc-treatment. There were 155 DEGs regulated by both ERDL4-overexpression and Glc. D) Correlation between relative expression (based on log2-fold-change) of 155 DEGs between 35S-ERDL4 (against WT) and Glc (against Man). r = Pearson coefficient calculated from ERDL4/WT and Glc/Man matrices.

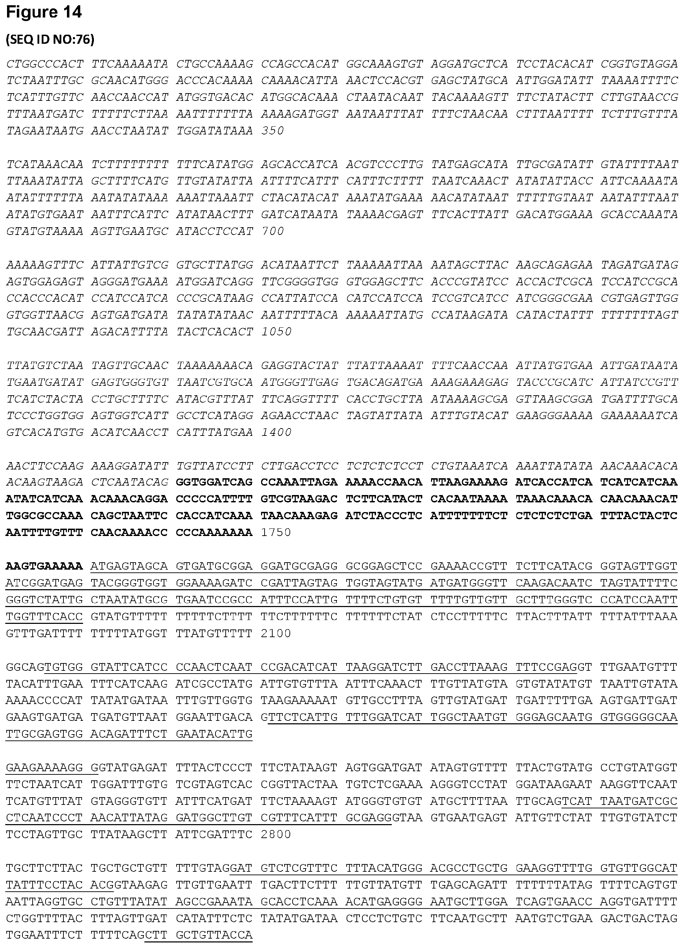

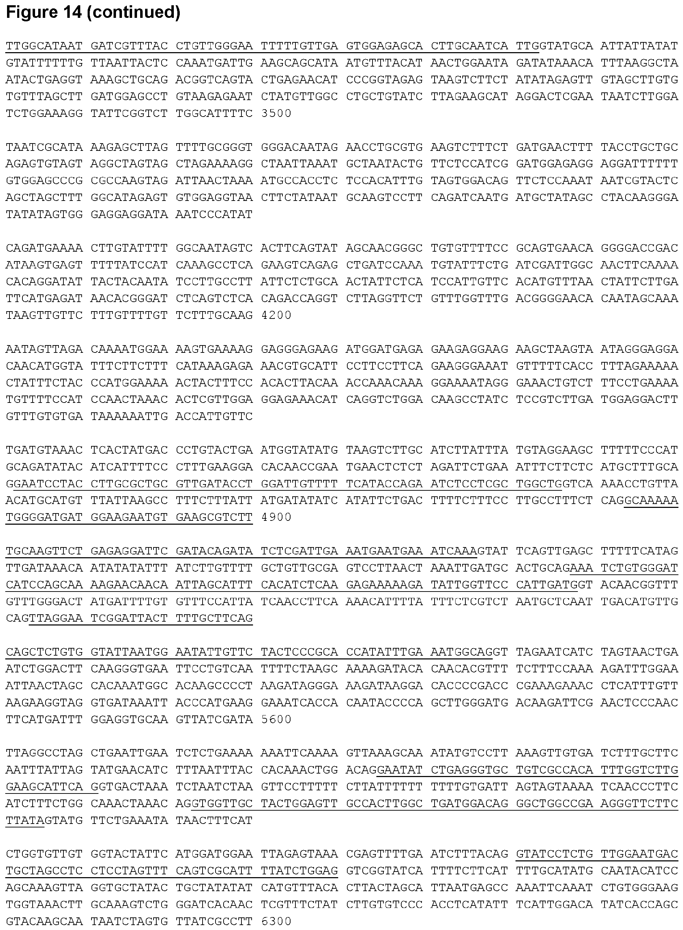

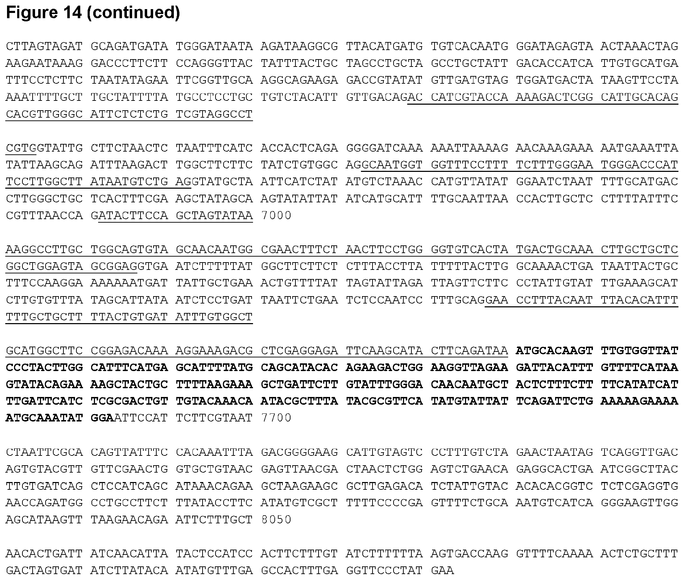

**Figure 14.** Figure 14 depicts the genomic sequence of the Beta vulgaris gene of ERDL4 (BvERDL4 ;2 (BvIMP2; Bv6_128840_qhip.t1), SEQ ID NO:76). The promoter region is depicted in italics, the 5'-UTR and 3'-UTR regions are given in boldface, the CDS regions are underlined.

## DETAILED DESCRIPTION OF THE INVENTION

All publications, including but not limited to patents, patent applications and scientific publications, cited in this description are herein incorporated by reference for all purposes as if each individual publication were specifically and individually indicated to be incorporated by reference.

Unless defined otherwise, all technical and scientific terms used herein have the meaning commonly understood by the person skilled in the art.

As used herein, the singular forms "a," "an" and "the" are intended to include the plural forms as well, unless the context clearly indicates otherwise.

As used herein, "and/or" refers to and encompasses any and all possible combinations of one or more of the associated listed items, as well as the lack of combinations when interpreted in the alternative ("or").

The use of the term "comprising" as well as other grammatical forms such as "comprises" and "comprised" is not limiting. The terms "comprising", "comprises" and "comprised" should be understood as referring to an open-ended description of an embodiment of the present invention that may, but does not have to, include additional technical features, in addition to the explicitly stated technical features. In the same sense, the term "involving" as well as other respective grammatical forms such as "involves" and "involved" is not limiting. The same applies for the term "including" and other grammatical forms such as "includes" and "included".

Section headings throughout the description are for organizational purposes only. In particular, they are not intended as limiting for various embodiments described therein, and it is to be understood that embodiments (and features therein) described under one subheading may be freely combined with embodiments (and features therein) described under another subheading.

Further, the terms "comprising", "involving" and "including", and any grammatical forms thereof, are not to be interpreted to exclusively refer to embodiments that include additional features to those explicitly recited. These terms equally refer to embodiments that consist of only those features that are explicitly mentioned.

As used herein, the term "nucleic acid" refers to an oligomer or polymer of naturally occurring or modified nucleotides. The nucleic acid may also comprise nucleotide substitutions relative to a reference sequence. For instance, the nucleic acid may contain 1, 2, 3, 4, 5, 6, 7, 8, 9 or 10 nucleotide substitutions. The nucleic acid may have 100 %, 95%, 90%, 85%, 80%, 75%, 70%, 65%, 60%, 55%, or 50% sequence identity to a reference sequence. A reference sequence may be a nucleic acid with a sequence according to SEQ ID NO: 1 to SEQ ID NO: 152. A nucleic acid may be a ribonucleic acid (RNA) or a deoxyribonucleic acid (DNA) or a hybrid between RNA and DNA. The nucleic acid may be single or double stranded. The double stranded nucleic acid may be RNA-RNA, RNA-DNA (hybrid) or DNA-DNA. Double strand formation occurs through for example, hydrogen bonds according to the Watson-Crick-base pairing. The nucleic acid may have one or two 3' overhangs. The nucleic acid may have one or two 5' overhangs. The nucleic acid may have one or two blunt ends. Combinations of the above-referenced overhangs or blunt ends are also included.

The nucleic acid can be defined by a sequence of nucleotides indicated in the commonly accepted letter code for the base of the nucleotide, A (adenine), C (cytosine), G (guanine), T (thymine) and U (uracil). "Sequence" as used herein refers to a sequence of nucleotides or a sequence of amino acids.

A sequence of amino acids may have 100 %, 95%, 90%, 85%, 80%, 75%, 70%, 65%, 60%, 55%, or 50% sequence identity to a reference sequence and/or may contain amino acid substitution relative to a reference sequence.

As used herein, the term "hybridizes under stringent conditions" means capable of hybridizing under stringent conditions, for instance, under conditions that correspond to a Tm (melting temperature) equal to or greater than about 50°C, 51°C, 53°C,54°C, 55°C, 56°C, 57°C, 58°C, 59°C, 60°C, 61°C, 62°C, 63°C, 64°C, 65°C, 66°C, 67°C, 68°C, 69°C

**EP 4 209 502 A1**

or 70°C.

An "increase in sucrose concentration" or an "increased sucrose concentration" or a "higher sucrose concentration of a sucrose storage organ of a plant" means an increase in the average sucrose concentration, based on the fresh weight of the sucrose storage organ, as compared with a reference plant cultured under identical conditions of at least 0.2%, 0.4%, 0.6%, 0.8% or 1%, preferably of at least 1.2%, 1.4%, 1.6%, 1.8% or 2%, particularly preferably of at least 2.5%, 3%, 3.5%, 4%, 4.5%, 5%, 6%, 7%, 8%, or 10%, and most preferably of at least 15%.

In some embodiments, the increase in yield of a plant storage compound is an increase in sucrose concentration.

"Increasing the yield of a plant storage compound" means that the yield is increased relative to a reference plant. The reference plant may be a reference plant as defined herein, for instance a wild type plant from which the plant with increased yield is derived. The plant with increased yield may be genetically modified, for instance the plant may comprise the vector or mobile genetic element of the invention. Yield of plant storage compound is determined as common in the art. For instance, yield may be mass of plant storage compound per area of cultivation.

A storage organ is a plant organ that stores plant storage compound. The storage organ may be a sugar storage organ. A "sugar storage organ" as used herein is a part of a plant, in which the plant stores sugar, such as sucrose and which may be harvested. In particular, sugar storage organs may be beets or fruits, such as corn kernel.

The "plant" may be a crop plant, in particular sugar beet (Beta vulgaris), cabbage (Brassica rapa), oilseed rape (Brassica napus), sunflower (Helianthus annuus), potato (Solanum tuberosum), corn (Zea mays), rice (Oryza sp., e.g. Oryza sativa), barley (Hordeum vulgare), rye (Secale cereale) or sorghum (Sorghum sp., e.g. Sorghum bicolor). In some embodiments, the plant is a transgenic plant. In some embodiments, the plant has been modified, in particular genetically modified. In some embodiments the plant modification leads to an increased level of fructose in the cytosol of the plant, in particular relative to a reference plant, such as a wild type plant from which the modified plant is derived. The increase of the fructose level may be achieved by overexpressing a tonoplast proton-fructose symporter, such as any one of the tonoplast proton-fructose symporter taught herein.

The term "overexpresses a tonoplast proton-fructose symporter" means that the amount of tonoplast proton-fructose symporter in the plant, plant cell or tonoplast is higher than in a reference plant, plant cell or tonoplast that has not been modified to overexpress. For instance, if a plant overexpresses a tonoplast proton-fructose symporter relative to the wild type plant from which it is derived, then the plant comprises a higher amount of tonoplast proton-fructose symporter in the plant, plant cell or tonoplast than the wild type plant, plant cell or tonoplast.

A "tonoplast proton-fructose symporter" is a co-transporter protein that is located in the tonoplast and transports protons and fructose from the vacuole into the cytosol.

The invention includes a vector or mobile genetic element, comprising a nucleic acid molecule, wherein the nucleic acid molecule encodes a tonoplast proton-fructose symporter and wherein the nucleic acid molecule is selected from the group consisting of:

a) a nucleic acid molecule having at least 50% nucleic acid sequence identity with a nucleic acid sequence selected from the group consisting of any one of the sequences according to SEQ ID NO: 40, 39, 1, and 129-152,

b) a nucleic acid molecule having a nucleic acid sequence that is complementary to or hybridizes under stringent conditions with the nucleic acid molecule according to a),

c) a nucleic acid molecule having a nucleic acid sequence that encodes a protein having at least 50% amino acid sequence identity with an amino acid sequence selected from the group consisting of any one of the sequences according to SEQ ID NO: 58, 57, 22, and 77-128.

In one embodiment the nucleic acid molecule comprised in the vector or mobile genetic element has at least 55%, at least 60%, at least 65%, at least 70%, at least 75%, at least 80%, at least 85%, at least 90%, at least 95%, or 100% sequence identity with a nucleic acid sequence selected from the group consisting of any one of the sequences according to SEQ ID NO: 40, 39, 1, and 129-152.

In one embodiment the protein encoded by the nucleic acid molecule has at least 55%, at least 60%, at least 65%, at least 70%, at least 75%, at least 80%, at least 85%, at least 90%, at least 95%, or 100% sequence identity with an amino acid sequence selected from the group consisting of any one of the sequences according to SEQ ID NO: 58, 57, 22, and 77-128.

The vector or mobile genetic element may further comprise one or more of any one of the nucleic acid sequences according to SEQ ID NO: 1-152 or a nucleic acid molecule having at least 50%, at least 55%, at least 60%, at least 65%, at least 70%, at least 75%, at least 80%, at least 85%, at least 90%, at least 95% sequence identity with a nucleic acid sequence selected from the group consisting of any one of the nucleic acid sequences according to SEQ ID NO: 1-152.

The vector or mobile genetic element may further comprise one or more nucleic acid sequences encoding any one of the amino acid sequences according to SEQ ID NO: 1-152 or one or more nucleic acid sequences encoding an

7

amino acid sequence having at least 50%, at least 55%, at least 60%, at least 65%, at least 70%, at least 75%, at least 80%, at least 85%, at least 90%, at least 95% sequence identity with an amino acid sequence selected from the group consisting of any one of the amino acid sequences according to SEQ ID NO: 1-152.

The vector or mobile genetic element may be used in the methods and uses taught herein, for example to achieve an overexpression of the tonoplast proton-fructose symporter. For instance, the vector or mobile genetic element may be used to produce a host cell as described herein, so that the host cell, for instance a plant cell, comprises the vector or mobile genetic element. The vector or mobile genetic element may be used to produce a plant as described herein. In particular, the plant described herein may comprise the vector or mobile genetic element. The vector or mobile genetic element may be used to increase the yield of a plant storage compound. The vector or mobile genetic element may be used to increase the biomass of a plant, in particular the biomass of at least on organ of the plant. The vector or mobile genetic element may be used to increase the concentration of at least one plant storage compound in at least one organ of the plant. In some embodiments, the yield of the plant storage compound may be increased by increasing the biomass of at least one organ of the plant, or by increasing the concentration of the plant storage compound in at least one plant organ, or a combination of both increasing the biomass and the concentration. Further, the vector or mobile genetic element may be used to increase the sucrose concentration in a sucrose storage organ of a plant as described herein, in particular by using the vector or mobile genetic element in a method of increasing the sucrose concentration in a sucrose storage organ of a plant as described herein. The vector or mobile genetic element may also be applied in the described use of a tonoplast proton-fructose symporter in a plant to increase the concentration of sucrose in a sucrose storage organ of the plant, for instance by introducing the vector or mobile genetic element into a cell of the plant. The vector or mobile genetic element may also find use in the described method of increasing the biomass of a plant. In particular, overexpressing the tonoplast proton-fructose symporter as described herein as encoded by the nucleic acid molecule comprised in the vector or mobile genetic element leads to an increased production of biomass of the plant that comprises the vector or mobile genetic element. The vector or mobile genetic element, when comprised in a plant, also leads to an increase in sucrose concentration as described herein.

The vector or mobile genetic element may be introduced into a cell, such as a eukaryotic or prokaryotic cell, by transformation, transfection or other means known in the art. For instance, the vector or mobile genetic element may be introduced into a plant cell by infecting the plant cell with Agrobacterium tumefaciens, which contains the vector or mobile genetic element. The vector or mobile genetic element may also be introduced into a plant cell by biolistic transfer or protoplast transformation. Preferably, the vector or mobile genetic element is stably maintained in the cell by incorporating the vector or mobile genetic element into the genome of the cell. Stable incorporation of the vector or mobile genetic element into the genome of the cell, such as a plant cell, allows regenerating a plant from the cell stably carrying the vector or mobile genetic element, thereby producing a transgenic plant.

Genome editing may be used to introduce the vector or mobile genetic element into the plant genome. Genome editing may be used to modify the promotor of an endogenous gene encoding a tonoplast proton-fructose symporter so that the tonoplast proton-fructose symporter is overexpressed. Genome editing includes CRSIPR gene editing, for instance using CRISPR- nuclease systems.

In a further aspect, the invention is directed to a host cell comprising the vector or mobile genetic element. A host cell may be a cell as described therein, in particular a plant cell of a plant as described herein.

In a further aspect, the invention is directed to a plant or part of the plant comprising at least one host cell, wherein the host cell preferably comprises a vector or mobile genetic element as described herein.

The plant described herein is in particular characterized by being genetically modified so that the plant comprises elevated levels of fructose in the cytosol relative to the wild type plant from which it is derived. Fructose levels in the cytosol of the plant may be at least 5%, at least 10%, at least 15%, at least 20%, at least 25%, at least 30%, at least 35%, at least 40%, at least 45%, at least 50%, at least 60%, at least 70%, at least 80%, at least 90%,at least 100%, at least 110%, at least 120%, at least 130%, at least 140%, at least 150%, at least 160%, at least 170%, at least 180%, at least 190%, at least 200%, at least 250%, at least 300%, at least 350%, at least 400%, at least 450%, or at least 500% higher than fructose levels in the cytosol of the wild type plant.

Elevated fructose levels in the cytosol of a plant, for instance a transgenic plant, may be achieved in different ways. For instance, elevated levels of fructose in the cytosol of a plant may be achieved in any way that leads to an overexpression of tonoplast proton-fructose symporter, in particular the tonoplast proton-fructose symporter disclosed herein.

Overexpression of the tonoplast proton-fructose symporter can be for instance achieved by activating the promoter of an endogenous tonoplast proton-fructose symporter gene. The TATA box of the promoter may be modified to achieve overexpression. Cis-regulatory elements may be introduced, for instance via genome editing as described herein and as known to the skilled person, to achieve overexpression. Promotor activation in plants can be achieved as described for instance in EP 3 546 582.

In some embodiments, overexpression of the tonoplast proton-fructose symporter is achieved by introducing at least one additional copy of a tonoplast proton-fructose symporter into the plant cell. The additional copy of the tonoplast proton-fructose symporter may be an additional copy of an endogenous tonoplast proton-fructose symporter or an additional copy of a heterologous tonoplast proton-fructose symporter.

In some embodiments, the tonoplast proton-fructose symporter may be any one of the tonoplast proton-fructose symporter disclosed herein, for instance ERDL4 of Arabidopsis thaliana (At) (SEQ ID NO: 1 and SEQ ID NO: 22), BvIMP of Beta vulgaris (Bv) (SEQ ID NO: 39 and SEQ ID NO: 57), BvIMP2 of Beta vulgaris (SEQ ID NO: 40 and SEQ ID NO: 58), or any one of the sequences according to SEQ ID NO: 77-128. Preferably, the tonoplast proton-fructose symporter may be any one of the candidates identified in the examples herein. More preferably, the tonoplast proton-fructose symporter may be ERDL4 of Arabidopsis thaliana, BvIMP of Beta vulgaris or BvIMP2 of Beta vulgaris.

In a particularly preferred embodiment, the plant is a Beta vulgaris that overexpresses a tonoplast proton-fructose symporter, such as BvIMP or BvIMP2.

In some embodiments, the tonoplast proton-fructose symporter comprises an amino acid sequence that has at least 50%, at least 55%, at least 60%, at least 65%, at least 70%, at least 75%, at least 80%, at least 85%, at least 90%, at least 95%, or 100% sequence identity with any one of the tonoplast proton-fructose symporter amino acid sequences taught herein, in particular SEQ ID NO: 58, 57, 22, and 77-128.

In addition to overexpressing a tonoplast proton-fructose symporter, other means can also be applied to increase the level of fructose in the cytosol, to achieve the beneficial effects of the present invention.

For instance, down regulation of the expression of SWEET17 (Sugars Will Eventually be Exported Transporter 17) may also increase the amount of fructose in the cytosol. SWEET17 is capable of transporting fructose across the tonoplast. In some embodiments, overexpression of a tonoplast proton-fructose symporter may be combined with down regulation of the expression of SWEET17. Down regulating the expression of SWEET17 may be achieved by various means, for instance genetically knocking out the SWEET17 gene or using RNAi. In some embodiments SWEET17 is SWEET17 of Arabidopsis thaliana (SEQ ID NO: 5, 28) or SWEET17 of Beta vulgaris (SEQ ID NO: 45, 64). The down regulation of SWEET17 can be applied to any plant, in particular crop plant that has an ortholog of SWEET17.

Fructose levels in the cytosol may also be increased by reducing the activity of fructose kinases and/or hexokinases that phosphorylate fructose and reduce the amount of free fructose in the cytosol. Therefore, it is envisaged to reduce the expression level of fructokinases and/or hexokinases (e.g. by down regulation or knockout) to increase fructose levels in the cytosol. In some embodiments, the fructokinases and/or hexokinases are selected from the group of fructokinases and/or hexokinases disclosed herein. Preferably, the reduction of the expression level of fructokinases and/or hexokinases may be combined with overexpressing the tonoplast proton-fructose symporter and/or down regulating the expression of SWEET17.

In some embodiments, TILLING (Targeting Induced Local Lesions in Genomes), using a chemical mutagen such as ethyl methanesulfonate (EMS), may be applied to identify mutant plants that possess increased cytosolic levels of fructose. For instance, in some embodiments TILLING may be used to identify plants that overexpress a tonoplast proton-fructose symporter, such as ERDL4, BvIMP or BvIMP2.

Increasing the yield of a plant storage compound refers to increasing the yield of the plant storage compound relative to a corresponding plant that is not a plant as taught herein, in particular a corresponding plant with lower fructose levels in the cytosol and/or a plant that has not been modified according to the present technical teaching. A corresponding plant is a plant of the same species.

The methods and uses of the invention, for instance the method of producing a plant, the method of increasing the yield of a plant storage compound and the method of increasing drought resistance in plants may include a step of growing the plant.

## EXAMPLES

**Example 1:** Overexpression of ERDL4 increases fructose in the cytosol

The over-expression of a tonoplast-located transporter called ERDL4 (Early Response to Dehydration-Like 4), a proton-symporter belonging to a large sub-group of the MST family of transporters (Fig.1; Büttner, 2007) that exports fructose from the vacuole to the cytosol (Fig.2 and 3), leads to an increase in fructose in the cytosol. Whether other sugars are also transported remains to be elucidated.

To be sensed and by that to contribute to nuclear signaling, fructose must be present in the cytosol. Otherwise, transcriptional regulation would not occur as the vacuole can be regarded 'outside' a cell, and the nucleus is blind to outside the cell. In a non-aqueous fractionation (NAF) experiment it could be shown that in ERDL4 over-expressing Arabidopsis thaliana plants fructose content is specifically increased in the cytosol (Fig.9).

Generation of ERDL4 Oex. Lines:
Full-length coding region of ERDL4 was amplified with primers ERDL4_Fwd: (5'-ggggacaagtttgtacaaaaaagcaggcttaATGAGTTTTAGGGATGATAATACG-3') and ERDL4_Rev: (5'-ggggaccactttgtacaagaaagctgggtaTCATCTGAACAAAGCTTGGATCTC-3') and cloned into pDON/Zeo and subcloned into pK2GW7 GatewayTM vector (Karimi et al., 2002) to create overexpression lines in Col-0 background. Agrobacterium tumefaciens strain GV3101 was used for transformation. Arabidopsis Col-0 plants were transformed via floral dip method (Clough and Bent, 1998). Positive lines were screened by Kanamycin antibiotic resistance and RT-PCR was used further for selection of strongest lines.

Metabolite quantification

Extraction of soluble sugars was done with 1ml of 80% Ethanol added to 100 mg of frozen finely ground plant material at 80°C for 30 min. The extracts were collected and evaporated in a vacufuge concentrator (Eppendorf, Hamburg, Germany). Resulting pellets were dissolved in ddH2O and quantified using NADP-coupled enzymatic test (Stitt et al., 1989) via 96-well plate reader (Tecan Infinite 200; Tecan Group).

**Example 2:** Increase of cytosolic fructose is beneficial for plant biomass production.

Arabidopsis thaliana ERDL4 over-expressor plants have a bigger leaf rosette (Fig.4) as well as higher root biomass (Fig.5), the 1000 seed weight is higher (Fig.6) and the lipid content of seeds higher than in control plants (Fig.7). In addition, sugar flux exported from leaves via the phloem is higher than in control plants (Fig.8).

Increased rosette size of ERDL4 over-expressors, root biomass and seed weight:
Rosette size was determined by measuring the fresh weight of the rosettes from the four-week-old plants grown on soil substrate ED73 (Patzer Einheitserde, Sinntal-Altengronau, Germany) under short days (10 h light/ 14 h dark) at 21°C. Rosettes were cut above the hypocotyl and cleaned of soil particles.

Root biomass was determined from four-week-old plants grown in a hydroponic system. Plants were removed from the hydroponic system and roots of individual plants cut below the hypocotyl. The severed root stocks were turned back and forth on a paper towel to remove excess water. Root fresh weight was determined using a precision balance (Sartorius, Göttingen, Germany). After determination of the fresh weight, roots were dried in an oven at 55°C overnight and weighed again.

For determination of the seed weight, plants from the different lines were grown for five weeks under short days (10 h light/ 14 h dark) at 21°C and then transferred to long days (18 h light/ 6 h dark) at 21°C until seed maturation. Around 1000 mature seeds of at least four plants from WT, the two ERDL4-overexpressing and the two erdl4 mutant lines were counted and then weighed. The 1000-seed weight was calculated by dividing the weight of the harvested seeds by the number of seeds and multiplying the value with 1000.

Determination of lipid content:
Lipid quantification was done according to previously optimized protocols (Reiser et al., 2004). 100 mg of seeds were homogenized in a mortar with liquid nitrogen. 1.5 ml of isopropanol was added to the homogenate and seeds were further homogenized. It was then transferred to 1.5 ml Eppendorf tubes and incubated at 4°C for 12 hours at 100 rpm. Samples were centrifuged at 13,000 g for 10 minutes. Subsequently, supernatant was transferred to pre-weighed 1.5 ml Eppendorf tubes and allowed to evaporate at 60°C for 8 hours. The remaining lipid pellet was quantified gravimetrically.

Phloem exudate measurements:
Phloem exudates were collected according to the protocol from Xu et al. [2019] with few modifications. Source leaves of same developmental stage from 6 weeks old well-watered plants, were cut with a sharp razor blade at the base of the petiole. Petioles were dipped in 20mM K2-EDTA solution. Leaves of the same line were stacked and the petioles were recut while dipped in the EDTA solution. Leaves were then transferred to 1.5 ml Eppendorf tubes with 500 ul of EDTA solution. Exudates were collected for 6 hours in a dark and humid chamber. Leaves were weighed and exudates were dried in a vacuum concentrator. For sugar quantification pellets were solubilized in 100 ul of ddH2O.

**Example 3:** Increase of cytosolic fructose can be sensed in the nucleus and triggers transcriptional regulation

ERDL4 over-expression increases cytosolic fructose which is sensed by the nucleus. The transcription of some genes is de-regulated through the presence of increased fructose in the cytosol. One example of such a gene is CIPK6 (Calcineurin B-like protein (CBL) interacting protein kinase 6; Fig.10) the protein of which activates TST2 (Tonoplast Sugar Transporter 2) through phosphorylation which is then importing more monosaccharides into the vacuole (Deng et al., 2020). Whether this activation of TST2 is part of the explanation for biomass increase through fructose sensing remains to be elucidated. Over-expressing CIPK6 would be a possibility to test this. On the other hand, a knock-out of CIPK6 in ERDL4 over-expressors would probably lead to a dissection of transcriptional and post-translational activation of TST2 and might give a hint whether any of these activations or which one is crucial for the ERDL4 over-expression phenotype. However, over-expression of a TST in Arabidopsis thaliana has been shown to result in a similar phenotype with increased biomass (Wormit et al., 2006; Wingenter et al., 2010). Other genes are also deregulated in ERDL4 over-expressors and/or in high cytosolic fructose plants. These could also be part of the explanation for the apparent phenotype.

For RNA-Seq analysis, RNA was isolated from source leaves of six-week old plants grown under short days (10 h light, 14 h dark at 21°C) on soil substrate ED73 (Einheitserde Patzer, Sinntal-Altengronau, Germany) using the RNEasy KIT (Qiagen, Hilden, Germany). Shortly, about 100 mg of frozen and ground tissue were extracted with 1.5 ml Qiazol Lysis reagent in 2 ml Eppendorf cups and centrifuged at 4°C at 12.000 g. Supernatants were purified using RNEasy spin colums. RNA concentration was calculated using a Nanodrop 2000/2000c (Thermo Fisher, Germany). About five micrograms of total RNA were shipped to NOVOGENE, (Cambridge, UK) for RNA library construction and bioinformatics RNAseq analysis (https://en.novogene.com).

**Example 4:** Increase of cytosolic fructose by reducing fructokinase and hexokinase activities for increased plant biomass production

Moreover, several enzymes exist that phosphorylate fructose to F6P in order to funnel back fructose into carbon metabolism - a reduction of activities of certain fructokinases (FrcKs) and hexokinases (HxKs) brought about by knock out or down-regulation of expression might therefore also lead to higher fructose levels and by that to fructose sensing and an increased plant biomass production.

**Example 5:** Over-expression of ERDL4 and down-regulation of SWEET17 gives synergistic effects and leads to increased cytosolic fructose levels for increased plant biomass production

In addition, SWEET17 (Sugars Will Eventually be Exported Transporter 17) of Arabidopsis is capable of fructose transport across the tonoplast. However, as SWEET17 is a facilitator rather than an activated transporter, fructose cannot be accumulated in the cytosol (Chardon et al., 2013; Guo et al., 2014; Klemens et al., 2014; Valifard et al., 2021). A potentially existing fructose gradient across the tonoplast (concentration of fructose in vacuole vs. cytosol) can only be equated. Therefore, a concomitant over-expression of ERDL4 and a down-regulation of SWEET17 is expected to lead to even higher cytosolic fructose levels. E.g. for sugar beet, BvERDL4;2 (sequence see appendix) and BvSWEET17 (the sequence of the closest ortholog of SWEET17 from Arabidopsis thaliana is given in the appendix) might be modified to increase cytosolic fructose.

**Example 6**: Overexpression of ERDL4 homologs in crop plants for increased plant biomass production

- Overexpression using a transgene

As an example for a crop we intend to improve, envisaged experiments will be described for sugar beet. However, the improvement of other crops is also envisaged. Overexpression of the sugar beet ortholog of Arabidopsis thaliana ERDL4 is expected to lead to increased biomass and yield. It is likely that BvERDL4;2 (BvIMP2) is the respective ortholog with relevant function as BvIMP2 overexpression leads to fructose export from the vacuole (Experiment 7; Fig.11). Expression of BvIMP2 under control of a constitutive (e.g. the 35S-CaMV promoter or a ubiquitin promoter) or under a leaf- or green tissue-specific promoter (e.g. a chlorophyll a/b binding protein or the StLS1 promoter, respectively) is envisaged to mimick the Arabidopsis thaliana ERDL4 overexpressor phenotype.

- Overexpression by promoter activation of the endogenous gene

An alternative to the GM approach described above is to activate the promoter of BvIMP2 by modifying core promoter elements like the TATA box or by introducing cis-regulatory elements via genome editing. Such a modified promoter would then lead to a higher expression of BvIMP2 which - in turn - would lead to the phenotype described above.

- TILLING mutants leading to ERDL4 overexpression

Moreover, an activation of the promoter can also be brought about by mutagenesis, e.g. single point mutations improving a core promoter element. In addition, mutagenesis of the coding sequence or other adjacent regulatory sequences (not determined yet) can lead to an improved activity enhancing fructose export from the vacuole.

- Mutagenesis of BvERDL4;1 to gain fructose transport function

As the sequence of BvERDL4;1 (BvIMP) is similar to BvIMP2, it seems reasonable that its function can be rendered towards fructose transport by mutagenesis. Such an extra fructose exporter could lead to an increase of cytosolic fructose and to a similar phenotype described above.

- Over-expression of ERDL4 and in addition down-regulation/knock out of SWEET17

Similar to what is described above, an overexpression of BvIMP2 in the background of a sweet17 mutant (brought about by mutagenesis or genome editing) can lead to an even higher cytosolic fructose content (see experiment 4). Alternatively, BvIMP2 overexpression and RNAi against BvSWEET17 can be combined to reach this aim.

- Screening populations of plants for natural over-expressors of ERDL4 with subsequent increase of the allele in a breeding pool

Populations of sugar beet (and other crops) elite or exotic breeding material will be screened for genotypes with increased expression of BvIMP2. Such screening will be done via RNAseq of leaves. Detection of natural overexpressors will be used for crossing into elite material to increase the respective allele in the breeding pool. This should ultimately lead to increased yield.

**Example 7:** Overexpression of potential sugar beet ERDL4 homolog in Arabidopsis shows fructose export from the vacuole

When analyzing the course of sugar and starch contents in Arabidopsis thaliana (Col-0, attst1-2, BvIMP2 OX1 and OX2) under cold stress (4°C) and re-acclimation (20°C; Fig.11), the sugar beet ortholog of AtERDL4, BvIMP2 (Bv6_128840_qhip.t1), could be assigned vacuolar fructose export function. Contents of glucose (a), fructose (b), sucrose (c) and starch (d) are shown over 14 days. Fructose content of BvIMP2 OX1 and OX2 does not reach the level of fructose in control plants, and it decreases earlier than in control plants. This is indicative of BvIMP2 to export fructose from the vacuole (in addition to the endogenous AtERDL4). Fructose in the cytosol could then be funneled into metabolism and used for anabolic reactions. Here, the extra fructose seems to be a precursor for building up starch.

For analysis of sugar and starch contents in Arabidopsis thaliana (Col-0, attst1-2, BvIMP2 OX1 and OX2) under cold stress (4°C) and re-acclimation (20°C) plants were cultivated on standardized soil (ED-73; DIN1154080T) mixed with 151 sand on 501 soil. Prior to the experiment plants were grown at 20°C (10h light/14 dark) at 110$\mu$mol m-2 s-1 for 21 days. After three weeks plants were transferred to 4°C at same light/dark regime. Plant material was harvested prior to 4°C treatment, after one, two, four and seven days after transfer to 4°C. After 4°C treatment for full seven days remaining plants were transferred back to 20°C and plant material was harvested four and seven days after retransfer. Four biological replicates each consisting of a pool of 4 plant rosettes were harvested at 10am immediately after onset of light. The harvested plant material was transferred to liquid nitrogen, pulverized, and stored at -80°C until use.

Soluble sugars were extracted from frozen material. For this purpose, about 50 mg of powdered plant material was weighed into 1 ml of 80% EtOH solution and extracted with 1 ml of 80% EtOH solution at 80°C for one hour with shaking at 350 rpm twice. After each extraction step, the insoluble components were centrifuged at 14,000 rpm for ten minutes and their supernatant was combined in 2 ml reaction tubes. The combined metabolite extracts were then evaporated in a Concentrator plus Vacofuge (Eppendorf, Hamburg, Germany). The evaporated pellets were dissolved in 1 ml ddH2O and stored at -20°C until measurement. The pellets remaining after extraction were washed with 500 $\mu$l of 80% EtOH and 1 ml of ddH2O and used to extract starch.

For extraction of starch the washed pellets remaining after extraction of soluble metabolites were mixed with 200 $\mu$l ddH2O and then autoclaved at 121°C for 40 min. Starch chains present in the pellet were then hydrolytically digested. For hydrolytic cleavage of starch, 200 $\mu$l enzyme mix (5 U $\alpha$-amylase, Sigma-Aldrich, Munich, Germany; 5 U amyloglucosidase, Roche, Mannheim, Germany; 200 mM sodium acetate; pH 4.8) was added to the autoclaved pellet and incubated at 37°C for at least four hours. To stop the enzyme reactions, the sample was then heated to 95°C for ten minutes. After final centrifugation at 14,000 rpm for ten minutes, the supernatant was used to quantify starch.

Concentrations of sugars (glucose, fructose, sucrose) and hydrolyzed starch were measured by NAD+-coupled enzymatic assay as described by Stitt et al., 1989. For this purpose, 190 $\mu$l of premix was added to 10 -20 $\mu$l of sample. Glucose, fructose, and sucrose were measured photometrically at a wavelength of 340 nm in an Infinite® M Nano microplate reader (Tecan, Männedorf, Switzerland) after addition of the enzymes hexokinase (Roche, Mannheim, Germany), phosphoglucoisomerase (Roche, Mannheim, Germany), and invertase (Sigma-Aldrich, Munich, Germany). The sugar content was then calculated according to Lambert-Beer's law.

Composition of the premix for sugar quantification: HEPES (pH 7.5) 100mM, MgCl2 10mM, ATP 2mM, NADP 1mM and Glucose-6-phosphate dehydrogenase from Leuconostoc mesenteroides 0.5U.

**Table 1.** Analyzed lines

| Name | Line | Genetic background | Modification | Reference |
|---|---|---|---|---|
| *attst1-2* | *tst1-2::tDNA* | Col-0 | Crossing of tmt1::tDNA and tmt2::tDNA | (Wormit et al., 2006) |

(continued)

| Name | Line | Genetic background | Modification | Reference |
|------|------|--------------------|--------------| ----------|
| *BvIMP2 OX1* | *35S::BvIMP2* #1-8 | Col-0 | 35S-BvIMP2cds | (Zierer, Wolfgang; FAU Erlangen) |
| *BvIMP2 OX2* | *35S::BvIMP2* #4-5 | Col-0 | 35S-BvIMP2cds | |

**Example 8:** Overexpression of ERDL4 for increased drought tolerance

ERDL4 over-expressor Arabidopsis plants have a higher biomass already under well-watered control conditions (100% field capacity; compare Fig.4). When subjecting the plants to mild (60% field capacity) or harsher (40% field capacity) drought stress, fresh weight biomass decreases. However, the decrease of biomass was less pronounced in over-expressor plants than in controls (Fig.12). Calculating the fresh weight ratio of over-expressors and controls demonstrates this:

100% field capacity – Oex1/Wt =1.38; Oex2/Wt = 1.29

60% field capacity – Oex1/Wt = 1.72; Oex2/Wt = 2.01

40% field capacity – Oex1/Wt = 1.66; Oex2/Wt = 1.49

The beneficial effect of ERDL4 over-expression for drought tolerance can be explained by improved root growth (compare Fig.5) of over-expressor plants which can use their extended root system to forage for water better than control plants or even erdl4 mutants (KO1, KO2). Consequently, mutants with smaller root system are particularly sensitive to drought.

Application of drought stress on plants grown on soil substrate:

Plants were exposed to drought conditions based on soil field capacity (FC) (Bouzid et al., 2019). Plants were kept at the determined water content on soil (control=100%, 60%, 40% FC) by a 48h interval of irrigation until harvesting. Seeds of wild types and ERDL4 overexpressing lines were germinated on standard soil (ED-73; Einheitserde Patzer; Sinntal-Altengronau, Germany) and plants were grown for one week prior to transfer to soil with 60% moisture/field capacity (FC) as compared to the control soil (100%). To prevent osmotic shock, seedlings were not directly transferred from 100% to 40% FC. Instead, two-week-old seedlings which were grown for one week at 100% and one week at 60% FC, were not watered until 40% moisture in the soil was reached. Afterwards, the level of soil moisture was kept constant by monitoring pot weight (with a precision balance and an accuracy of 0.01g) in 48h intervals (Xt). To calculate the field capacity of the soil, three pots were filled with the corresponding soil, followed by drying in an oven for 4 days at 60 °C to determine the weight of dry soil (X0) in each pot. Consequently, three further pots were filled with the same amount of soil as described for drying followed by watering the soil until water saturation (Xf). Subsequent to this, pots were transferred to the growth chamber. After 48h, pots were weighed again (Xt). The percentage for field capacity of the soil was calculated using the following formula; $[(Xt-X0)/(Xf-X0)] *100$ (Bouzid et al., 2019).

Drought stress was applied based on the field capacity of the soil (100%, 60%, 40%). Plants subjected to stress after 7 days of germination. Stress was applied for 3.5 weeks. Fresh weights were determined by taking the rosette fresh weight immediately after harvest.

**Example 9:** Many genes de-regulated in Arabidopsis thaliana ERDL4 overexpressors are also de-regulated in fructose-floated rather than glucose-floated Arabidopsis thaliana leaf discs

Overexpression of ERDL4 results in elevated fructose and glucose levels in the cytosol and it is reasonable to check for consequences on global gene expression caused by the altered intracellular sugar distribution by means of RNASeq analysis. To test whether changes of the transcriptome of ERDL4-overexpresors were caused by intracellular glucose or fructose accumulation, control experiments were performed in which discs excised from mature Arabidopsis leaves were incubated overnight in 0.5 MS buffer solution pH 5.7 supplemented with either 1% glucose, 1% fructose or 1% mannitol (as osmotic control). Under these conditions, the supplemented sugars diffuse via the apoplasm to intact mesophyll cells where they are imported into the cytosol. Such flooding with pure sugar species has been shown to evoke sugar species-specific responses on the level of gene expression (Wormit et al., 2006; Luo et al., 2012). Genes which exhibit significant differential expression between different plant lines or between different treatments are named "differentially expressed genes" (DEGs). DEGs between ERDL4-overexpressors and wild-type (WT) have been identified by testing for significant differences of the expression of individual genes by double-sided t-test. DEGs between fructose and mannitol incubation or between glucose and mannitol incubation have been identified by testing for significant differences of the expression of individual genes by double-sided t-test. With conditional analysis DEGs overlapping in ERDL4-overexpressors and fructose treatment or DEGs overlapping in ERDL4-overexpressors and glucose treatment were identified (Figure 13). In total, 130 common DEGs between ERDL4-overexpressors and fructose, and 155 common DEGs between ERDL4-overexpressors and glucose were identified (Figure 13). Correlation analysis based on the individual amplitudes of gene expression changes of the 130 common DEGs from ERDL4-overexpressors and fructose and of the 155 common DEGs from ERDL4-overexpressors and glucose treatments revealed Pearson coefficients of r = 0.48 (r$^2$ = 0.23) and r = 0.15 (r$^2$ = 0.0225) for ERDL4-overexpressors and fructose treatment and ERDL4-overexpressors and glucose treatment, respectively. The analysis revealed a ten-times higher correlation between ERDL4-overexpressing- and fructose-dependent gene expression changes when compared to ERDL4-overexpressing- and glucose-dependent gene expression and indicated that the transcriptional changes recorded in ERDL4-overexpressors were caused by altered fructose rather than altered glucose signaling.

**Example 10:** Identification of orthologous sequences to AtERDL4 in several dicotyledonous and monocotyledonous crop species

In order to identify crop genes orthologous to the Arabidopsis thaliana ERDL4 gene, a series of sequence comparisons was carried out using the respective protein (amino acid) sequences of these genes. Genes/proteins of several crops were analyzed: Sugar beet (Beta vulgaris), cabbage (Brassica rapa), oilseed rape (Brassica napus), sunflower (Helianthus annuus), potato (Solanum tuberosum), corn (Zea mays), rice (Oryza sativa), barley (Hordeum vulgare), rye (Secale cereale) and sorghum (Sorghum bicolor). To demonstrate that the Arabidopsis thaliana proteins can be distinguished from each other using a BLAST search (Altschul et al., 1997; with standard conditions: Expect: 10; Matrix: BLOSUM62 producing pairwise alignments), a comparison amongst AtERDL4, 6 and 7 was carried out first (Table 2, also compare Fig.1). The higher the score the more similar the sequences are. The reason to include ERDL6 and 7 into the analyses is that ERDL6 is the most similar paralog of ERDL4 in Arabidopsis thaliana, and ERDL7 is one of the second most similar but already quite different to ERDL4 and 6 (compare also Fig.1).

**Table 2:** Arabidopsis thaliana ERDL4, 6 and 7 comparison with each other

|          | AtERDL4 | AtERDL6 | AtERDL7 |
|----------|---------|---------|---------|
| AtERDL4  | **947** | 880     | 417     |
| AtERDL6  | 880     | **951** | 414     |
| AtERDL7  | 417     | 414     | 904     |

AtERDL4, 6 and 7 can be discriminated from each other.

In order to define putative orthologs of AtERDL4 in Brassica rapa, 3 BLAST searches were conducted using (i) AtERDL4, (ii) AtERDL6 and (iii) AtERDL7, respectively, as query sequences against protein sequences of the Brassica rapa genome. The resulting BLAST scores are listed in Table 3.

Table 3: Comparison of AtERDL4, AtERDL6 and AtERDL7, respectively, with Brassica rapa proteins

|      | AtERDL4 | AtERDL6 | AtERDL7 |
|------|---------|---------|---------|
| BrA  | **910** | 882     | 406     |
| BrB  | **904** | 866     | 405     |
| BrC  | 865     | **912** | 410     |
| BrD  | 860     | **903** | 412     |
| BrE  | 860     | **901** | 416     |
| BrF  | 414     | 411     | 856     |

BrA and BrB (see all sequences in the appendix) are candidates for AtERDL4 orthologs in cabbage, and BrC, BrD and BrE for AtERDL6. BrF is a candidate ortholog for AtERDL7. Additional sequences from Arabidopsis thaliana could be included (Fig.1) in the analyses. However, the purpose of sequence comparisons here is solely to define the most likely candidates for orthologs of AtERDL4 (and not of all AtERDL proteins).

In order to define putative orthologs of AtERDL4 in Brassica napus, 3 BLAST searches were conducted using (i) AtERDL4, (ii) AtERDL6 and (iii) AtERDL7, respectively, as query sequences against protein sequences of the Brassica napus genome. The resulting BLAST scores are listed in Table 4.

Table 4: Comparison of AtERDL4, AtERDL6 and AtERDL7, respectively, with Brassica napus proteins

|  | AtERDL4 | AtERDL6 | AtERDL7 |
|---|---|---|---|
| BnA | **911** | 884 | 408 |
| BnB | **910** | 882 | 406 |
| BnC | **907** | 870 | 405 |
| BnD | **901** | 865 | 407 |
| BnE | 863 | **905** | 413 |
| BnF | 862 | **910** | 409 |
| BnG | 862 | **904** | 413 |
| BnH | 861 | **902** | 417 |
| BnI | 724 | 759 | 325 |
| BnJ | 416 | 414 | 857 |

BnA, BnB, BnC and BnD are candidates for AtERDL4 orthologs in oilseed rape. BnE, BnF, BnG and BrH are more likely orthologs of AtERDL6. BnI and BnJ are likely no candidate orthologs for AtERDL4.

In order to define putative orthologs of AtERDL4 in Beta vulgaris, 3 BLAST searches were conducted using (i) AtERDL4, (ii) AtERDL6 and (iii) AtERDL7, respectively, as query sequences against protein sequences of the Beta vulgaris genome. The resulting BLAST scores are listed in Table 5.

Table 5: Comparison of AtERDL4, AtERDL6 and AtERDL7, respectively, with Beta vulgaris proteins

|  | AtERDL4 | AtERDL6 | AtERDL7 |
|---|---|---|---|
| BvIMP | **775** | **789** | 409 |
| BvIMP2 | **710** | **720** | 394 |
| BvC | 420 | 427 | 665 |

BvIMP and BvIMP2 are candidates for AtERDL4 (and AtERDL6) orthologs in sugar beet. BvC is likely no candidate ortholog for AtERDL4 or 6. These data are supported by functional analysis of BvIMP2, which indicates that BvIMP2 is a functional ortholog of AtERDL4 (see Fig. 11).

In order to define putative orthologs of AtERDL4 in Helianthus annuus, 3 BLAST searches were conducted using (i) AtERDL4, (ii) AtERDL6 and (iii) AtERDL7, respectively, as query sequences against protein sequences of the Helianthus annuus genome. The resulting BLAST scores are listed in Table 6.

Table 6: Comparison of AtERDL4, AtERDL6 and AtERDL7, respectively, with Helianthus annuus proteins

|  | AtERDL4 | AtERDL6 | AtERDL7 |
|---|---|---|---|
| HaA | **773** | **786** | 391 |
| HaB | **763** | **771** | 396 |
| HaC | 682 | 680 | 384 |
| HaD | 422 | 421 | 695 |

HaA and HaB are candidates for AtERDL4 (and AtERDL6) orthologs in sunflower. HaD is likely no candidate ortholog for AtERDL4 or 6.

In order to define putative orthologs of AtERDL4 in Solanum tuberosum, 3 BLAST searches were conducted using (i) AtERDL4, (ii) AtERDL6 and (iii) AtERDL7, respectively, as query sequences against protein sequences of the Solanum tuberosum genome. The resulting BLAST scores are listed in Table 7.

Table 7: Comparison of AtERDL4, AtERDL6 and AtERDL7, respectively, with Solanum tuberosum proteins

|     | AtERDL4 | AtERDL6 | AtERDL7 |
| --- | --- | --- | --- |
| StA | **780** | **779** | 408 |
| StB | **780** | **796** | 402 |
| StC | 714 | 723 | 412 |
| StD | 691 | 698 | 399 |
| StE | 575 | 583 | 334 |
| StF | 422 | 419 | 525 |

StA and StB are candidates for AtERDL4 (and AtERDL6) orthologs in potato. StF is likely no candidate ortholog for AtERDL4 or 6.

In order to define putative orthologs of AtERDL4 in several monocotyledonous species, 3 BLAST searches each were conducted using (i) AtERDL4, (ii) AtERDL6 and (iii) AtERDL7, respectively, as query sequences against protein sequences of the genome of several monocotyledonous species. The resulting BLAST scores are listed in the following tables. In order to define putative orthologs of AtERDL4 in Zea mays, 3 BLAST searches were conducted using (i) AtERDL4, (ii) AtERDL6 and (iii) AtERDL7, respectively, as query sequences against protein sequences of the Zea mays genome. The resulting BLAST scores are listed in the following Table 8.

Table 8: Comparison of AtERDL4, AtERDL6 and AtERDL7, respectively, with Zea mays proteins

|     | AtERDL4 | AtERDL6 | AtERDL7 |
| --- | --- | --- | --- |
| ZmA | **710** | **707** | 406 |
| ZmB | **689** | **684** | 397 |
| ZmC | 595 | 595 | 389 |
| ZmD | 594 | 603 | 381 |
| ZmE | 566 | 562 | 342 |
| ZmF | 424 | 413 | 488 |

ZmA and ZmB are best candidates for AtERDL4 (and AtERDL6) orthologs in corn. ZmF is no candidate ortholog for AtERDL4 or 6.

In order to define putative orthologs of AtERDL4 in Oryza sativa, 3 BLAST searches were conducted using (i) AtERDL4, (ii) AtERDL6 and (iii) AtERDL7, respectively, as query sequences against protein sequences of the Oryza sativa genome. The resulting BLAST scores are listed in Table 9.

Table 9: Comparison of AtERDL4, AtERDL6 and AtERDL7, respectively, with Oryza sativa proteins

|     | AtERDL4 | AtERDL6 | AtERDL7 |
| --- | --- | --- | --- |
| OsA | **720** | **710** | 397 |
| OsB | **691** | **692** | 374 |
| OsC | **658** | **654** | 374 |
| OsD | 408 | 410 | 570 |
| OsE | 367 | 377 | 514 |

(continued)

|  | AtERDL4 | AtERDL6 | AtERDL7 |
|---|---|---|---|
| OsF | 367 | 371 | 501 |

OsA, OsB and OsC are candidates for orthologs for AtERDL4 (and AtERDL6) in rice. OsD, OsE and OsF are less likely candidates for AtERDL4 (and AtERDL6) orthologs.

In order to define putative orthologs of AtERDL4 in Hordeum vulgare 3 BLAST searches were conducted using (i) AtERDL4, (ii) AtERDL6 and (iii) AtERDL7, respectively, as query sequences against protein sequences of the Hordeum vulgare genome. The resulting BLAST scores are listed in Table 10.

Table 10: Comparison of AtERDL4, AtERDL6 and AtERDL7, respectively, with Hordeum vulgare proteins

|  | AtERDL4 | AtERDL6 | AtERDL7 |
|---|---|---|---|
| HvA | **686** | **684** | 385 |
| HvB | **684** | **681** | 383 |
| HvC | **660** | **652** | 372 |
| HvD | 401 | 400 | 514 |

HvA, HvB or HvC are candidates for orthologs for AtERDL4 (and AtERDL6) in barley. HvD is no candidate for AtERDL4 (and AtERDL6) orthologs.

In order to define putative orthologs of AtERDL4 in Secale cereale, 3 BLAST searches were conducted using (i) AtERDL4, (ii) AtERDL6 and (iii) AtERDL7, respectively, as query sequences against protein sequences of the Secale cereale genome. The resulting BLAST scores are listed in Table 11.

Table 11: Comparison of AtERDL4, AtERDL6 and AtERDL7, respectively, with Secale cereale proteins

|  | AtERDL4 | AtERDL6 | AtERDL7 |
|---|---|---|---|
| ScA | **698** | **697** | 394 |
| ScB | **677** | **674** | 379 |
| ScC | **672** | **668** | 378 |
| ScD | 658 | 648 | 374 |
| ScE | 407 | 405 | 539 |

ScA, ScB or ScC are candidates for orthologs for AtERDL4 (and AtERDL6) in rye. ScD and particularly ScE are less likely candidates for AtERDL4 (and AtERDL6) orthologs.

In order to define putative orthologs of AtERDL4 in Sorghum bicolor, 3 BLAST searches were conducted using (i) AtERDL4, (ii) AtERDL6 and (iii) AtERDL7, respectively, as query sequences against protein sequences of the Sorghum bicolor genome. The resulting BLAST scores are listed in Table 12.

Table 12: Comparison of AtERDL4, AtERDL6 and AtERDL7, respectively, with Sorghum bicolor proteins

|  | AtERDL4 | AtERDL6 | AtERDL7 |
|---|---|---|---|
| SbA | **704** | **701** | 407 |
| SbB | **680** | **675** | 397 |
| SbC | **673** | **674** | 371 |
| SbD | 610 | 609 | 365 |
| SbE | 422 | 416 | 482 |

SbA, SbB or SbC are candidates for orthologs for AtERDL4 (and AtERDL6) in sorghum. SbD and particularly SbE are less likely candidates for AtERDL4 (and AtERDL6) orthologs.

The foregoing examples are illustrative of the present invention, and are not to be construed as limiting the invention to the embodiments disclosed therein. Although the invention has been described in detail with reference to preferred embodiments, variations and modifications exist within the scope and spirit of the invention as described and defined in the following claims.

References:

1) In concert: Orchestrated changes in carbohydrate homeostasis are critical for plant abiotic stress tolerance; Pommerrenig B et al. (2018), DOI: 10.1093/pcp/pcy037

2) ChloroP, a neural network based method for predicting chloroplast transit peptides and their cleavage sites; Emanuelsson O et al., (2008), DOI: 10.1110/ps.8.5.978

3) ARAMEMNON, a novel database for Arabidopsis integral membrane proteins; Schwacke R et al. (2003), DOI: 10.1104/pp.011577

4) Fast, scalable generation of high-quality protein multiple sequence alignments using Clustal Omega; Sievers F et al. (2011), DOI: 10.1038/msb.2011.75

5) MrBayes: Bayesian phylogenetic inference under mixed models; Ronquist F & Huelsenbeck JP (2003), DOI: 10.1093/bioinformatics/btg180

6) The monosaccharide transporter(-like) gene family in Arabidopsis; Büttner M (2007), DOI: 10.1016/j.febslet.2007.03.016

7) GATEWAY™ vectors for Agrobacterium-mediated plant transformation; Karimi M et al. (2002), DOI: 10.1016/S1360-1385(02)02251-3

8) "Floral dip: a simplified method for Agrobacterium-mediated transformation of Arabidopsis thaliana; Clough S & Bent AF (1998), DOI: 10.1046/j.1365-313x.1998.00343.x

9) Metabolite levels in specific cells and subcellular compartments of plant leaves; Stitt M (1989), DOI: 10.1016/0076-6879(89)74035-0

10) Molecular physiological analysis of the two plastidic ATP/ADP transporters from Arabidopsis; Reiser J et al (2004), DOI: 10.1104/pp.104.049502

11) Regulation of sucrose transporters and phloem loading in response to environmental cues; Xu Q et al (2018), DOI: 10.1104/pp.17.01088

12) The Calcium Sensor CBL2 and Its Interacting Kinase CIPK6 Are Involved in Plant Sugar Homeostasis via Interacting with Tonoplast Sugar Transporter TST2(1); Deng JW et al. (2020), DOI: 10.1104/pp.19.01368

13) Molecular identification and physiological characterization of a novel monosaccharide transporter from Arabidopsis involved in vacuolar sugar transport; Wormit A et al. (2006), DOI: 10.1105/tpc.106.047290

14) Increased Activity of the Vacuolar Monosaccharide Transporter TMT1 Alters Cellular Sugar Partitioning, Sugar Signaling, and Seed Yield in Arabidopsis; Wingenter K et al (2010), DOI: 10.1104/pp.110.162040

15) Leaf Fructose Content Is Controlled by the Vacuolar Transporter SWEET17 in Arabidopsis; Chardon F et al (2013), DOI: 10.1016/j.cub.2013.03.021

16) SWEET17, a Facilitative Transporter, Mediates Fructose Transport across the Tonoplast of Arabidopsis Roots and Leaves; Guo, WJ et al. (2014), DOI: 10.1104/pp.113.232751

17) Overexpression of a proton-coupled vacuolar glucose exporter impairs freezing tolerance and seed germination; Klemens, PAW et al. (2014), DOI: 10.1111/nph.12642

18) Vacuolar fructose transporter SWEET17 is critical for root development and drought tolerance; Valifard M et al (2021), DOI: 10.1093/plphys/kiab436

19) Arabidopsis species deploy distinct strategies to cope with drought stress; Bouzid M et al. (2019), DOI: 10.1093/aob/mcy237

20) An autoregulatory feedback loop involving PAP1 and TAS4 in response to sugars in Arabidopsis; Luo, QJ etal. (2012), DOI: 10.1007/s11103-011-9778-9

21) Gapped BLAST and PSI-BLAST: a new generation of protein database search programs; Altschul, SF et al. (1997), DOI: 10.1093/nar/25.17.3389

**Claims**

1. A vector or mobile genetic element, comprising a nucleic acid molecule, wherein the nucleic acid molecule encodes a tonoplast proton-fructose symporter and wherein the nucleic acid molecule is selected from the group consisting of:

   a) a nucleic acid molecule having at least 50% nucleic acid sequence identity with a nucleic acid sequence selected from the group consisting of any one of the sequences according to SEQ ID NO: 40, 39, 1, and 129-152,
   b) a nucleic acid molecule having a nucleic acid sequence that is complementary to or hybridizes under stringent conditions with the nucleic acid molecule according to a), and
   c) a nucleic acid molecule having a nucleic acid sequence that encodes a protein having at least 50% amino acid sequence identity with an amino acid sequence selected from the group consisting of any one of the sequences according to SEQ ID NO: 58, 57, 22, and 77-128.

2. A host cell comprising the vector or mobile genetic element according to claim 1.

3. A plant or a part of a plant comprising at least one host cell according to claim 1.

4. A plant, wherein the plant has been modified so that the plant comprises elevated levels of fructose in the cytosol relative to the wild type plant from which it is derived.

5. The plant of claim 4, wherein the plant overexpresses a tonoplast proton-fructose symporter relative to the wild type plant from which it is derived.

6. The plant of claim 5, wherein

   i) the plant comprises a modified promotor of an endogenous gene encoding the overexpressed tonoplast proton-fructose symporter so that the tonoplast proton-fructose symporter is homologously overexpressed,
   ii) the plant comprises at least one additional copy of a nucleic acid encoding the overexpressed tonoplast proton-fructose symporter so that the tonoplast proton-fructose symporter is homologously overexpressed, or
   iii) the plant comprises a heterologous nucleic acid encoding a heterologous tonoplast proton-fructose symporter so that the tonoplast proton-fructose symporter is heterologously overexpressed.

7. The plant of any one of claims 4 to 6, wherein the plant comprises the vector or mobile genetic element of claim 1.

8. A seed of a plant according to any one of claims 3 to 7.

9. A method of producing a plant, comprising the following steps:

   a) modifying at least one plant cell so that at least one plant cell is produced that comprises elevated levels of fructose in the cytosol relative to the wild type plant cell from which it is derived; and
   b) regenerating a plant from the modified plant cell of step a), wherein the plant comprises elevated levels of fructose in the cytosol relative to the wild type plant from which is derived.

10. The method of claim 9, wherein modifying the at least one plant cell in step a) comprises

i) modifying the promoter of an endogenous gene encoding a tonoplast proton-fructose symporter so that the tonoplast proton-fructose symporter is overexpressed,

ii) introducing into the plant cell at least one additional copy of a nucleic acid encoding a tonoplast proton-fructose symporter so that the tonoplast proton-fructose symporter is homologously overexpressed, or

iii) introducing into the plant cell a heterologous nucleic acid encoding a heterologous tonoplast proton-fructose symporter so that the tonoplast proton-fructose symporter is heterologously overexpressed.

11. The method of claim 9 or 10, wherein the vector or mobile genetic element of claim 1 is introduced into the at least one plant cell.

12. A method of increasing the yield of a plant storage compound, comprising increasing the concentration of fructose in the cytosol of the plant.

13. The method of claim 12, wherein

i) the plant that produces the plant storage compound is produced according to any one of claims 9 to 11, and/or

ii) the plant that produces the plant storage compound is a plant of any one of claims 3 to 7.

14. The method of any one of claims 12 or 13, wherein the plant storage compound is selected from a sugar, preferably sucrose, starch, a protein or a lipid.

15. The method of any one of claims 12 to 14, wherein

i) the biomass of at least one plant organ that comprises the plant storage compound is increased, preferably the plant organ is a fruit, a seed, a root or a storage organ, or

ii) the concentration of the plant storage compound in at least one plant organ is increased, preferably the plant organ is a fruit, a seed, a root or a storage organ.

16. A method of increasing drought tolerance in plants, comprising increasing the concentration of fructose in the cytosol of the plant.

17. The method of claim 16, wherein

i) the plant that has increased drought tolerance is produced according to any one of claims 9 to 11, and/or

ii) the plant that has increased drought tolerance is a plant of any one of claims 3 to 7.

18. The plant of any one of claims 3 to 7 and the method of any one of claims 9 to 17, wherein the plant is selected from the group consisting of sugar beet (Beta vulgaris), cabbage (Brassica rapa), oilseed rape (Brassica napus), sunflower (Helianthus annuus), potato (Solanum tuberosum), corn (Zea mays), rice (Oryza sp., e.g. Oryza sativa), barley (Hordeum vulgare), rye (Secale cereale) or sorghum (Sorghum sp.), preferably the plant is sugar beet.

19. The method of any one of claims 12 to 15, wherein the plant is sugar beet, the plant organ is the sugar beet taproot and the plant storage compound is sucrose.

20. Use of a tonoplast proton-fructose symporter in a plant to increase the concentration of a plant storage compound in a plant organ, optionally the plant storage compound is selected from a sugar, preferably sucrose, starch, a protein or a lipid, and the plant is selected from the group consisting of sugar beet (Beta vulgaris), cabbage (Brassica rapa), oilseed rape (Brassica napus), sunflower (Helianthus annuus), potato (Solanum tuberosum), corn (Zea mays), rice (Oryza sp., e.g. Oryza sativa), barley (Hordeum vulgare), rye (Secale cereale) or sorghum (Sorghum sp.), preferably the plant is sugar beet and the plant organ is the sugar beet taproot.

21. A method of selecting a plant with increased fructose levels in the cytosol of the plant, comprising the steps:

i) growing a plant;

ii) measuring the fructose level in at least one plant cell of the plant;

iii) comparing the measured fructose level to at least one fructose reference level; and

iv) selecting the plant with increased fructose levels relative to the at least one reference level.

Figure 1A

Figure 1B

**Figure 2**

**Figure 3**

**Figure 4**

A

B

C

**Figure 5**

**Figure 6**

Figure 7

Figure 8

Figure 9

Figure 10

**Figure 11**

Figure 12

**A**

**B**  *35S-ERDL4*/WT *vs* Frc/Man

**C**

**D**  *35S-ERDL4*/WT *vs* Glc/Man)

**Figure 13**

# Figure 14

**(SEQ ID NO:76)**

CTGGCCCACT TTCAAAAATA CTGCCAAAAG CCAGCCACAT GGCAAAGTGT AGGATGCTCA TCCTACACAT CGGTGTAGGA
TCTAATTTGC GCAACATGGG ACCCACAAAA CAAAACATTA AACTCCACGT GAGCTATGCA ATTGGATATT TAAAATTTTC
TCATTTGTTC AACCAACCAT ATGGTGACAC ATGGCACAAA CTAATACAAT TACAAAAGTT TTCTATACTT CTTGTAACCG
TTTAATGATC TTTTTCTTAA AATTTTTTTA AAAAGATGGT AATAATTTAT TTTCTAACAA CTTTAATTTT TCTTTGTTTA
TAGAATAATG AACCTAATAT TGGATATAAA 350

TCATAAACAA TCTTTTTTTT TTTCATATGG AGCACCATCA ACGTCCCTTG TATGAGCATA TTGCGATATT GTATTTTAAT
TTAAATATTA GCTTTTCATG TTGTATATTA ATTTTCATTT CATTTCTTTT TAATCAAACT ATATATTACC ATTCAAAATA
ATATTTTTTA AATATATAAA AAATTAAATT CTACATACAT AAATATGAAA AACATATAAT TTTTTGTAAT AATATTTAAT
ATATGTGAAT AATTTCATTC ATATAACTTT GATCATAATA TAAAACGAGT TTCACTTATT GACATGGAAA GCACCAAATA
GTATGTAAAA AGTTGAATGC ATACCTCCAT 700

AAAAAGTTTC ATTATTGTCG GTGCTTATGG ACATAATTCT TAAAAATTAA AATAGCTTAC AAGCAGAGAA TAGATGATAG
AGTGGAGAGT AGGGATGAAA ATGGATCAGG TTCGGGGTGG GTGGAGCTTC ACCCGTATCC ACCACTCGCA TCCATCCGCA
CCACCCACAT CCATCCATCA CCCGCATAAG CCATTATCCA CATCCATCCA TCCGTCATCC ATCGGGCGAA CGTGAGTTGG
GTGGTTAACG AGTGATGATA TATATATAAC AATTTTTACA AAAAATTATG CCATAAGATA CATACTATTT TTTTTTTAGT
TGCAACGATT AGACATTTTA TACTCACACT 1050

TTATGTCTAA TAGTTGCAAC TAAAAAAACA GAGGTACTAT TTATTAAAAT TTTCAACCAA ATTATGTGAA ATTGATAATA
TGAATGATAT GAGTGGGTGT TAATCGTGCA ATGGGTTGAG TGACAGATGA AAAGAAAGAG TACCCGCATC ATTATCCGTT
TCATCTACTA CCTGCTTTTC ATACGTTTAT TTCAGGTTTT CACCTGCTTA ATAAAAGCGA GTTAAGCGGA TGATTTTGCA
TCCCTGGTGG AGTGGTCATT GCCTCATAGG AGAACCTAAC TAGTATTATA ATTTGTACAT GAAGGGAAAA GAAAAAATCA
GTCACATGTG ACATCAACCT CATTTATGAA 1400

AACTTCCAAG AAAGGATATT TGTTATCCTT CTTGACCTCC TCTCTCTCCT CTGTAAATCA AAATTATATA AACAAACACA
ACAAGTAAGA CTCAATACAG **GGTGGATCAG CCAAATTAGA AAAACCAACA TTAAGAAAAG ATCACCATCA TCATCATCAA**
**ATATCATCAA ACAAACAGGA CCCCCATTTT GTCGTAAGAC TCTTCATACT CACAATAAAA TAAACAAACA CAACAAACAT**
**TGGCGCCAAA CAGCTAATTC CACCATCAAA TAACAAAGAG ATCTACCCTC ATTTTTTTCT CTCTCTCTGA TTTACTACTC**
**AATTTTGTTT CAACAAAACC CCCAAAAAAA** 1750

**AAGTGAAAAA** ATGAGTAGCA GTGATGCGGA GGATGCGAGG GCGGAGCTCC GAAAACCGTT TCTTCATACG GGTAGTTGGT
ATCGGATGAG TACGGGTGGT GGAAAAGATC CGATTAGTAG TGGTAGTATG ATGATGGGTT CAAGACAATC TAGTATTTTC
GGGTCTATTG CTAATATGCG TGAATCCGCC ATTTCCATTG TTTTCTGTGT TTTTGTTGTT GCTTTGGGTC CCATCCAATT
TGGTTTCACC GTATGTTTTT TTTTTCTTTT TTCTTTTTTC TTTTTTCTAT CTCCTTTTTC TTACTTTATT TTTATTTAAA
GTTTGATTTT TTTTTATGGT TTATGTTTTT 2100

GGCAGTGTGG GTATTCATCC CCAACTCAAT CCGACATCAT TAAGGATCTT GACCTTAAAG TTTCCGAGGT TTGAATGTTT
TACATTTGAA TTTCATCAAG ATCGCCTATG ATTGTGTTTA ATTTCAAACT TTGTTATGTA GTGTATATGT TAATTGTATA
AAAACCCCAT TATATGATAA TTTGTTGGTG TAAGAAAAAT GTTGCCTTTA GTTGTATGAT TGATTTTTGA AGTGATTGAT
GAAGTGATGA TGATGTTAAT GGAATTGACA GTTCTCATTG TTTGGATCAT TGGCTAATGT GGGAGCAATG GTGGGGGCAA
TTGCGAGTGG ACAGATTTCT GAATACATTG

GAAGAAAAGG GGTATGAGAT TTTACTCCCT TTCTATAAGT AGTGGATGAT ATAGTGTTTT TTACTGTATG CCTGTATGGT
TTCTAATCAT TGGATTTGTG TCGTAGTCAC CGGTTACTAA TGTCTCGAAA AGGGTCCTAT GGATAAGAAT AAGGTTCAAT
TCATGTTTAT GTAGGGTGTT ATTTCATGAT TTCTAAAAGT ATGGGTGTGT ATGCTTTTAA TTGCAGTCAT TAATGATCGC
CTCAATCCCT AACATTATAG GATGGCTTGT CGTTTCATTT GCGAGGGTAA GTGAATGAGT ATTGTTCTAT TTGTGTATCT
TCCTAGTTGC TTATAAGCTT ATTCGATTTC 2800

TGCTTCTTAC TGCTGCTGTT TTTGTAGGAT GTCTCGTTTC TTTACATGGG ACGCCTGCTG GAAGGTTTTG GTGTTGGCAT
TATTTCCTAC ACGGTAAGAG TTGTTGAATT TGACTTCTTT TTGTTATGTT TGAGCAGATT TTTTTTATAG TTTTCAGTGT
AATTAGGTGC CTGTTTATAT AGCCGAAATA GCACCTCAAA ACATGAGGGG AATGCTTGGA TCAGTGAACC AGGTGATTTT
CTGGTTTTAC TTTAGTTGAT CATATTTCTC TATATGATAA CTCCTCTGTC TTCAATGCTT AATGTCTGAA GACTGACTAG
TGGAATTTCT TTTTCAGCTT GCTGTTACCA

**Figure 14 (continued)**

```
TTGGCATAAT GATCGTTTAC CTGTTGGGAA TTTTTGTTGA GTGGAGAGCA CTTGCAATCA TTGGTATGCA ATTATTATAT
GTATTTTTTG TTAATTACTC CAAATGATTG AAGCAGCATA ATGTTTACAT AACTGGAATA GATATAAACA TTTAAGGCTA
ATACTGAGGT AAAGCTGCAG ACGGTCAGTA CTGAGAACAT CCCGGTAGAG TAAGTCTTCT ATATAGAGTT GTAGCTTGTG
TGTTTAGCTT GATGGAGCCT GTAAGAGAAT CTATGTTGGC CTGCTGTATC TTAGAAGCAT AGGACTCGAA TAATCTTGGA
TCTGGAAAGG TATTCGGTCT TGGCATTTTC 3500

TAATCGCATA AAGAGCTTAG TTTTGCGGGT GGGACAATAG AACCTGCGTG AAGTCTTTCT GATGAACTTT TACCTGCTGC
AGAGTGTAGT AGGCTAGTAG CTAGAAAAGG CTAATTAAAT GCTAATACTG TTCTCCATCG GATGGAGAGG AGGATTTTTT
GTGGAGCCCG CGCCAAGTAG ATTAACTAAA ATGCCACCTC TCCACATTTG TAGTGGACAG TTCTCCAAAT AATCGTACTC
AGCTAGCTTT GGCATAGAGT GTGGAGGTAA CTTCTATAAT GCAAGTCCTT CAGATCAATG ATGCTATAGC CTACAAGGGA
TATATAGTGG GAGGAGGATA AATCCCATAT

CAGATGAAAA CTTGTATTTT GGCAATAGTC ACTTCAGTAT AGCAACGGGC TGTGTTTTCC GCAGTGAACA GGGGACCGAC
ATAAGTGAGT TTTTATCCAT CAAAGCCTCA GAAGTCAGAG CTGATCCAAA TGTATTTCTG ATCGATTGGC AACTTCAAAA
CACAGGATAT TACTACAATA TCCTTGCCTT ATTCTCTGCA ACTATTCTCA TCCATTGTTC ACATGTTTAA CTATTCTTGA
TTCATGAGAT AACACGGGAT CTCAGTCTCA CAGACCAGGT CTTAGGTTCT GTTTGGTTTG ACGGGGAACA CAATAGCAAA
TAAGTTGTTC TTTGTTTTGT TCTTTGCAAG 4200

AATAGTTAGA CAAAATGGAA AAGTGAAAAG GAGGGAGAAG ATGGATGAGA GAAGAGGAAG AAGCTAAGTA ATAGGGAGGA
CAACATGGTA TTTCTTCTTT CATAAAGAGA AACGTGCATT CCTTCCTTCA GAAGGGAAAT GTTTTTCACC TTTAGAAAAA
CTATTTCTAC CCATGGAAAA ACTACTTTCC ACACTTACAA ACCAAACAAA GGAAAATAGG GAAACTGTCT TTCCTGAAAA
TGTTTTCCAT CCAACTAAAC ACTCGTTGGA GGAGAAACAT CAGGTCTGGA CAAGCCTATC TCCGTCTTGA TGGAGGACTT
GTTTGTGTGA TAAAAAATTG ACCATTGTTC

TGATGTAAAC TCACTATGAC CCTGTACTGA ATGGTATATG TAAGTCTTGC ATCTTATTTA TGTAGGAAGC TTTTTCCCAT
GCAGATATAC ATCATTTTCC CTTTGAAGGA CACAACCGAA TGAACTCTCT AGATTCTGAA ATTTCTTCTC ATGCTTTGCA
GGAATCCTAC CTTGCGCTGC GTTGATACCT GGATTGTTTT TCATACCAGA ATCTCCTCGC TGGCTGGTCA AAACCTGTTA
ACATGCATGT TTATTAAGCC TTTCTTTATT ATGATATATC ATATTCTGAC TTTTCTTTCC TTGCCTTTCT CAGGCAAAAA
TGGGGATGAT GGAAGAATGT GAAGCGTCTT 4900

TGCAAGTTCT GAGAGGATTC GATACAGATA TCTCGATTGA AATGAATGAA ATCAAAGTAT TCAGTTGAGC TTTTTCATAG
TTGATAAACA ATATATATTT ATCTTGTTTT GCTGTTGCGA GTCCTTAACT AAAATTGATGC ACTGCAGAAA TCTGTGGGAT
CATCCAGCAA AAGAACAACA ATTAGCATTT CACATCTCAA GAGAAAAAGA TATTGGTTCC CATTGATGGT ACAACGGTTT
GTTTGGGACT ATGATTTTGT GTTTCCATTA TCAACCTTCA AAACATTTTA TTTCTCGTCT AATGCTCAAT TGACATGTTG
CAGTTAGGAA TCGGATTACT TTTGCTTCAG

CAGCTCTGTG GTATTAATGG AATATTGTTC TACTCCCGCA CCATATTTGA AATGGCAGGT TAGAATCATC TAGTAACTGA
ATCTGGACTT CAAGGGTGAA TTCCTGTCAA TTTTCTAAGC AAAAGATACA CAACACGTTT TCTTTCCAAA AGATTTGGAA
ATTAACTAGC CACAAATGGC ACAAGCCCCT AAGATAGGGA AAGATAAGGA CACCCCGACC CGAAAGAAAC CTCATTTGTT
AAGAAGGTAG GTGATAAATT ACCCATGAAG GAAATCACCA CAATACCCCA GCTTGGGATG ACAAGATTCG AACTCCCAAC
TTCATGATTT GGAGGTGCAA GTTATCGATA 5600

TTAGGCCTAG CTGAATTGAA TCTCTGAAAA AAATTCAAAA GTTAAAGCAA ATATGTCCTT AAAGTTGTGA TCTTTGCTTC
AATTTATTAG TATGAACATC TTTAATTTAC CACAAACTGG ACAGGAATAT CTGAGGGTGC TGTCGCCACA TTTGGTCTTG
GAAGCATTCA GGTGACTAAA TCTAATCTAA GTTCCTTTTT CTTATTTTTT TTTTGTGATT AGTAGTAAAA TCAACCCTTC
ATCTTTCTGG CAAACTAAAC AGGTGGTTGC TACTGGAGTT GCCACTTGGC TGATGGACAG GGCTGGCCGA AGGGTTCTTC
TTATAGTATG TTCTGAAATA TAACTTTCAT

CTGGTGTTGT GGTACTATTC ATGGATGGAA TTAGAGTAAA CGAGTTTTGA ATCTTTACAG GTATCCTCTG TTGGAATGAC
TGCTAGCCTC CTCCTAGTTT CAGTCGCATT TTATCTGGAG GTCGGTATCA TTTTCTTCAT TTTGCATATG CAATACATCC
AGCAAAGTTA GGTGCTATAC TGCTATATAT CATGTTACA CTTACTAGCA TTAATGAGCC AAATTCAAAT CTGTGGGAAG
TGGTAAACTT GCAAAGTCTG GGATCACAAC TCGTTTCTAT CTTGTGTCCC ACCTCATATT TCATTGGACA TATCACCAGC
GTACAAGCAA TAATCTAGTG TTATCGCCTT 6300
```

**Figure 14 (continued)**

CTTAGTAGAT GCAGATGATA TGGGATAATA AGATAAGGCG TTACATGATG TGTCACAATG GGATAGAGTA ACTAAACTAG
AAGAATAAAG GACCCTTCTT CCAGGGTTAC TATTTACTGC TAGCCTGCTA GCCTGCTATT GACACCATCA TTGTGCATGA
TTTCCTCTTC TAATATAGAA TTCGGTTGCA AGGCAGAAGA GACCGTATAT GTTGATGTAG TGGATGACTA TAAGTTCCTA
AAATTTTGCT TGCTATTTTA TGCCTCCTGC TGTCTACATT GTTGACAG<u>AC CATCGTACCA AAAGACTCGG CATTGCACAG</u>
<u>CACGTTGGGC ATTCTCTCTG TCGTAGGCCT</u>

<u>CGTG</u>GTATTG CTTCTAACTC TAATTTCATC ACCACTCAGA GGGGATCAAA AAATTAAAAG AACAAGAAA AATGAAATTA
TATTAAGCAG ATTTAAGACT TGGCTTCTTC TATCTGTGGC AGG<u>CAATGGT GGTTTCCTTT TCTTTGGGAA TGGGACCCAT</u>
<u>TCCTTGGCTT ATAATGTCTG AGG</u>TATGCTA ATTCATCTAT ATGTCTAAAC CATGTTATAT GGAATCTAAT TTTGCATGAC
CTTGGGCTGC TCACTTTCGA AGCTATAGCA AGTATATTAT ATCATGCATT TTGCAATTAA CCACTTGCTC CTTTTATTTC
CGTTTAACCA <u>GATACTTCCA GCTAGTATAA</u> 7000

<u>AAGGCCTTGC TGGCAGTGTA GCAACAATGG CGAACTTTCT AACTTCCTGG GGTGTCACTA TGACTGCAAA CTTGCTGCTC</u>
<u>GGCTGGAGTA GCGGAGGTGA</u> ATCTTTTTAT GGCTTCTTCT CTTTACCTTA TTTTTACTTG GCAAAACTGA TAATTACTGC
TTTCCAAGGA AAAAAATGAT TATTGCTGAA ACTGTTTTAT TAGTATTAGA TTAGTTCTTC CCTATTGTAT TTGAAAGCAT
CTTGTGTTTA TAGCATTATA ATCTCCTGAT TAATTCTGAA TCTCCAATCC TTTGCAG<u>GAA CCTTTACAAT TTACACATTT</u>
<u>TTTGCTGCTT TTACTGTGAT ATTTGTGGCT</u>

<u>GCATGGCTTC CGGAGACAAA AGGAAAGACG CTCGAGGAGA TTCAAGCATA CTTCAGATAA</u> **ATGCACAAGT TTGTGGTTAT**
**CCCTACTTGG CATTTCATGA GCATTTTATG CAGCATACAC AGAAGACTGG AAGGTTAGAA GATTACATTT GTTTTCATAA**
**GTATACAGAA AAGCTACTGC TTTTAAGAAA GCTGATTCTT GTATTTGGGA CAACAATGCT ACTCTTTCTT TTCATATCAT**
**TTGATTCATC TCGCGACTGT TGTACAAACA ATACGCTTTA TACGCGTTCA TATGTATTAT TCAGATTCTG AAAAAGAAAA**
**ATGCAAATAT GGA**ATTCCAT TCTTCGTAAT 7700

CTAATTCGCA CAGTTATTTC CACAAATTTA GACGGGGAAG CATTGTAGTC CCTTTGTCTA GAACTAATAG TCAGGTTGAC
AGTGTACGTT GTTCGAACTG GTGCTGTAAC GAGTTAACGA CTAACTCTGG AGTCTGAACA GAGGCACTGA ATCGGCTTAC
TTGTGATCAG CTCCATCAGC ATAAACAGAA GCTAAGAAGC GCTTGAGACA TCTATTGTAC ACACACGGTC TCTCGAGGTG
AACCAGATGG CCTGCCTTCT TTATACCTTC ATATGTCGCT TTTTCCCCGA GTTTTCTGCA AATGTCATCA GGGAAGTTGG
AGCATAAGTT TAAGAACAGA ATTCTTTGCT 8050

AACACTGATT ATCAACATTA TACTCCATCC ACTTCTTTGT ATCTTTTTTA AGTGACCAAG GTTTTCAAAA ACTCTGCTTT
GACTAGTGAT ATCTTATACA ATATGTTTGA GCCACTTTGA GGTTCCCTAT GAA

Europäisches
Patentamt

European
Patent Office

Office européen
des brevets

# EUROPEAN SEARCH REPORT

Application Number

EP 22 15 0603

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 109 750 048 A (UNIV NORTHWEST A&F) 14 May 2019 (2019-05-14) | 1-11,21 | INV. C07K14/415 C12N15/82 |
| Y | * claims; figures 14,15; examples; sequence 1 * | 1-21 | |
| | ----- | | ADD. A01H5/00 |
| X | US 2011/179531 A1 (KOVALIC DAVID K [US] ET AL) 21 July 2011 (2011-07-21) | 21 | |
| Y | * paragraph [0026] – paragraph [0033] * | 1-21 | |
| | ----- | | |
| X | US 2017/037422 A1 (ALEXANDROV NICKOLAI [US] ET AL) 9 February 2017 (2017-02-09) | 21 | |
| Y | * paragraph [0554]; sequence 129558 * | 1-21 | |
| | ----- | | |
| Y | US 2007/118916 A1 (PUZIO PIOTR [DE] ET AL) 24 May 2007 (2007-05-24) * paragraph [0554]; sequence 57929 * | 1-21 | |
| | ----- | | |

TECHNICAL FIELDS SEARCHED (IPC)

C07K
C12N

The present search report has been drawn up for all claims

| Place of search | Date of completion of the search | Examiner |
|---|---|---|
| Munich | 20 June 2022 | Burkhardt, Peter |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
.................................................................
& : member of the same patent family, corresponding
document

EPO FORM 1503 03.82 (P04C01)

Europäisches
Patentamt

European
Patent Office

Office européen
des brevets

**Application Number**

**EP 22 15 0603**

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

**see sheet B**

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

**1-21(partially)**

☐ The present supplementary European search report has been drawn up for those parts of the European patent application which relate to the invention first mentioned in the claims (Rule 164 (1) EPC).

| | **LACK OF UNITY OF INVENTION** | **Application Number** |
|---|---|---|
| Europäisches Patentamt / European Patent Office / Office européen des brevets | **SHEET B** | **EP 22 15 0603** |

The Search Division considers that the present European patent application does not comply with the requirements of unity of invention and relates to several inventions or groups of inventions, namely:

```
        1. claims: 1-21(partially)

                The tonoplast proton-fructose symporter of SEQ ID NO 40, and
                methods and products comprising the said sequence
                                ---


        2-27. claims: 1-21(partially)

                As invention 1, but relating to SEQ ID NO 39, 1 and 129-152
                                ---
```

## ANNEX TO THE EUROPEAN SEARCH REPORT
## ON EUROPEAN PATENT APPLICATION NO.

EP 22 15 0603

This annex lists the patent family members relating to the patent documents cited in the above-mentioned European search report.
The members are as contained in the European Patent Office EDP file on
The European Patent Office is in no way liable for these particulars which are merely given for the purpose of information.

20-06-2022

| Patent document cited in search report | | Publication date | Patent family member(s) | | Publication date |
|---|---|---|---|---|---|
| CN 109750048 | A | 14-05-2019 | NONE | | |
| US 2011179531 | A1 | 21-07-2011 | US | 2011179531 A1 | 21-07-2011 |
| | | | US | 2013097737 A1 | 18-04-2013 |
| | | | US | 2015152146 A1 | 04-06-2015 |
| US 2017037422 | A1 | 09-02-2017 | US | 2017037422 A1 | 09-02-2017 |
| | | | US | 2018237794 A1 | 23-08-2018 |
| US 2007118916 | A1 | 24-05-2007 | AU | 2006219823 A1 | 08-09-2006 |
| | | | CA | 2598792 A1 | 08-09-2006 |
| | | | EP | 1777296 A2 | 25-04-2007 |
| | | | EP | 1871883 A1 | 02-01-2008 |
| | | | EP | 2175034 A2 | 14-04-2010 |
| | | | EP | 2194140 A2 | 09-06-2010 |
| | | | US | 2007118916 A1 | 24-05-2007 |
| | | | WO | 2006092449 A2 | 08-09-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3546582 A

**Non-patent literature cited in the description**

- **POMMERRENIG B et al.** *In concert: Orchestrated changes in carbohydrate homeostasis are critical for plant abiotic stress tolerance,* 2018
- **EMANUELSSON O et al.** *ChloroP, a neural network based method for predicting chloroplast transit peptides and their cleavage sites,* 2008
- **SCHWACKE R et al.** *ARAMEMNON, a novel database for Arabidopsis integral membrane proteins,* 2003
- **SIEVERS F et al.** *Fast, scalable generation of high-quality protein multiple sequence alignments using Clustal Omega,* 2011
- **RONQUIST F ; HUELSENBECK JP.** *MrBayes: Bayesian phylogenetic inference under mixed models,* 2003
- **BÜTTNER M.** *The monosaccharide transporter(-like) gene family in Arabidopsis,* 2007
- **KARIMI M et al.** *GATEWAY™ vectors for Agrobacterium-mediated plant transformation,* 2002
- **CLOUGH S ; BENT AF.** *Floral dip: a simplified method for Agrobacterium-mediated transformation of Arabidopsis thaliana,* 1998
- **STITT M.** *Metabolite levels in specific cells and subcellular compartments of plant leaves,* 1989
- **REISER J et al.** *Molecular physiological analysis of the two plastidic ATP/ADP transporters from Arabidopsis,* 2004
- **XU Q et al.** *Regulation of sucrose transporters and phloem loading in response to environmental cues,* 2018
- **DENG JW et al.** *The Calcium Sensor CBL2 and Its Interacting Kinase CIPK6 Are Involved in Plant Sugar Homeostasis via Interacting with Tonoplast Sugar Transporter TST2(1),* 2020
- **WORMIT A et al.** *Molecular identification and physiological characterization of a novel monosaccharide transporter from Arabidopsis involved in vacuolar sugar transport,* 2006
- **WINGENTER K et al.** *Increased Activity of the Vacuolar Monosaccharide Transporter TMT1 Alters Cellular Sugar Partitioning, Sugar Signaling, and Seed Yield in Arabidopsis,* 2010
- **CHARDON F et al.** *Leaf Fructose Content Is Controlled by the Vacuolar Transporter SWEET17 in Arabidopsis,* 2013
- **GUO, WJ et al.** *SWEET17, a Facilitative Transporter, Mediates Fructose Transport across the Tonoplast of Arabidopsis Roots and Leaves,* 2014
- **KLEMENS, PAW et al.** *Overexpression of a proton-coupled vacuolar glucose exporter impairs freezing tolerance and seed germination,* 2014
- **VALIFARD M et al.** *Vacuolar fructose transporter SWEET17 is critical for root development and drought tolerance,* 2021
- **BOUZID M et al.** *Arabidopsis species deploy distinct strategies to cope with drought stress,* 2019
- **LUO, QJ et al.** *An autoregulatory feedback loop involving PAP1 and TAS4 in response to sugars in Arabidopsis,* 2012
- **ALTSCHUL, SF et al.** *Gapped BLAST and PSI-BLAST: a new generation of protein database search programs,* 1997